(19) Europäisches Patentamt
European Patent Office
Office européen des brevets

(11) EP 2 100 594 A1

(12) EUROPÄISCHE PATENTANMELDUNG

(43) Veröffentlichungstag:
**16.09.2009 Patentblatt 2009/38**

(21) Anmeldenummer: **08152815.0**

(22) Anmeldetag: **14.03.2008**

(51) Int Cl.:
***A61K 8/97*** *(2006.01)* ***A61Q 19/08*** *(2006.01)*
***A61K 36/73*** *(2006.01)*

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **Symrise GmbH & Co. KG**
**37603 Holzminden (DE)**

(72) Erfinder:
- **Schmaus, Gerhard**
  **37671, Höxter (DE)**
- **Franke, Helge**
  **64807, Dieburg (DE)**
- **Lange, Sabine**
  **37603, Holzminden (DE)**

(74) Vertreter: **Eisenführ, Speiser & Partner**
**Patentanwälte Rechtsanwälte**
**Postfach 10 60 78**
**28060 Bremen (DE)**

(54) **Ternäre Mischungen mit Collagensynthese steigernder Wirkung**

(57) Die Erfindung betrifft synergistisch wirksame, die Collagensynthese steigernde ternäre Mischungen und deren Verwendungen, insbesondere zum Verlangsamen der Hautalterung und zur Wundheilung geschädigter Haut. Ferner betrifft die Erfindung die Verwendung der erfindungsgemäßen ternären Mischungen zur Behandlung des Mund- und Rachenraums, und dort insbesondere zur Vorbeugung und Verlangsamung von Paradontitis, zum Aufbau von parodontalem Bindegewebe sowie der Vorbeugung und Behandlung von Infektionen der Mundhöhle und der Wundheilung in der Mundhöhle.

EP 2 100 594 A1

Printed by Jouve, 75001 PARIS (FR)

## Beschreibung

Die Erfindung betrifft das Gebiet synergistisch wirksamer, die Collagensynthese steigernder ternärer Mischungen und deren Verwendungen insbesondere zu kosmetischen, mundhygienischen, und pharmazeutischen Zwecken. Im speziellen betrifft die Erfindung das Herstellen ternärer Mischungen enthaltend (a) mindestens einen Extrakt aus Aloe und (b) mindestens einen Stoff ausgewählt aus der Gruppe Ascorbinsäure und deren Derivate und (c) mindestens einen Extrakt aus einer Rosaceen Art (Rosaceae; Familie der Rosengewächse), vorzugsweise Himbeerblattextrakt, Erdbeerblattextrakt, Brombeerblattextrakt oder Mädesüßkrautextrakt. Weiterhin betrifft die Erfindung die Verwendung der ternären Mischungen zur Stimulation der Collagensynthese, insbesondere zum Zweck der Verlangsamung der Hautalterung, zur Reduktion von Faltenbildung, zur Behandlung entzündlicher Prozesse der Haut und zur Wundheilung geschädigter Haut. Ferner betrifft die Erfindung die Verwendung der erfindungsgemäßen ternären Mischungen zur Behandlung des Mund- und Rachenraums, insbesondere zur Vorbeugung und Verlangsamung von Paradontitis und dem Aufbau von parodontalem Bindegewebe sowie im weiteren der Vorbeugung und Behandlung von Infektionen der Mundhöhle und der Wundheilung in der Mundhöhle.

Die Alterung der menschlichen Haut geht einher mit zunehmender Faltenbildung und abnehmender Elastizität und Festigkeit. Beim Alterungsprozess wird unterschieden zwischen intrinsischer und extrinsischer Hautalterung. Die intrinsische Alterung umfasst die natürlichen Veränderungen der Haut, die durch genetische Vorgaben reguliert werden. Der Begriff extrinsische Hautalterung steht für vorzeitige Alterungsprozesse, die durch exogene Einflüsse wie Sonnenlicht, Umweltgifte (z. B. Ozon, Tabakrauch etc.), psychologischen Stress und chronische Entzündung hervorgerufen werden. Die ultraviolette Strahlung ist die wichtigste exogene Noxe, durch die es zu einer vorzeitigen Alterung der menschlichen Haut kommt (Lichtalterung). Neben dem natürlichen Sonnenlicht spielt hier die Bestrahlung der menschlichen Haut mit künstlicher UV-Strahlung (Solarium) eine immer größer werdende Rolle.

Die für das klinische Bild gealterter Haut verantwortlichen strukturellen Veränderungen finden vor allem in der Dermis statt. Elastizität und Festigkeit der Haut sind wesentlich durch die beiden Hauptbestandteile der dermalen extrazellulären Matrix, den beiden Faserproteinen Collagen und Elastin, bestimmt. Die Dermis enthält hauptsächlich Collagen-1 (90-85%), das ausschließlich von dermalen Fibroblasten gebildet wird, sowie in signifikant geringeren Mengen (10-15%) Collagen-3. Elastin, der Hauptbestandteil der elastischen Fasern der Haut neben Fibrillin, ist zu etwa 1-3% in der Dermis enthalten.

Alte Haut ist im Gegensatz zu junger Haut gekennzeichnet durch eine abnehmende Konzentration an Collagen und Elastin. Dieser altersbedingte Gewebeverlust ist durch die mit zunehmendem Alter einhergehende Verringerung der de novo Collagensynthese sowie durch ein Ungleichgewicht zwischen Aktivierung und Inhibition proteolytischer Aktivität begründet, der mit dem vermehrten Abbau von Collagen einhergeht.

Der Ausgleich der durch intrinsische und extrinsische Hautalterung hervorgerufenen Effekte wie reduzierte de novo Collagensynthese und erhöhte proteolytischen Aktivität der MMPs, insbesondere der MMP-1, -2, -3 und -9, ist somit ein wichtiges Ziel bei der Entwicklung neuer kosmetischer Wirkstoffe gegen Hautalterung und Falten.

Der Erhalt eines hohen Collagenspiegels der Haut bzw. die Erhöhung des Collagenspiegels der Haut kann auf verschiedenen Wegen erreicht werden. Einerseits können Stoffe eingesetzt werden, die Matrixmetalloproteinasen inhibieren. Andererseits können aber auch Stoffe eingesetzt werden, die die Collagensynthese steigern, um dadurch dem negativen Effekte des MMP induzierten Collagenabbaus durch de novo Synthese entgegenzuwirken. Die mit zunehmendem Alter einhergehende Verringerung der de novo Collagensynthese kann dabei durch den Einsatz von Wirkstoffen, die die Collagensynthese steigern zumindest teilweise kompensiert werden.

Häufig in Verbindung mit der Steigerung der Collagensynthese genannte Einzelstoffe und somit Stand der Technik sind zum Beispiel Wirkstoffe wie Ascorbinsäure und deren Derivate, Retinol sowie Abkömmlinge des Retinols oder Pflanzenextrakte wie zum Beispiel Extrakte aus Aloe- und Centella-Arten. Darüber hinaus zählen auch peptidische Stoffe und deren Derivate wie z.B. Carnitin, Carnosin, Kreatin, Matrikine Peptide (e.g. lysyl-threonyl-threonyl-lysyl-serine) sowie weitere peptidische Strukturen wie palmitoylierte Pentapeptide (z.B. Matrixyl/Fa. Sederma) oder das Oligopeptid mit dem Handelsnamen Vincipeptide (Fa. Vincience/Frankreich) zu häufig verwendeten, die Collagesynthese steigernden Wirkstoffen. Im weiteren finden Verbindungen wie Asiatsäure (Asiatic acid), Madecassic Säure, Madecassosid, Asiaticosid, Extrakte aus Centella asiatica, Niacinamid, , Astaxanthin, Glucane z.B aus Hefen und Hafer, Sojaextrakte sowie Sojaisoflavone wie Genistein und Daidzein, Rutin, Chrysin, Morin, Betelnuß-Alkaloide, Forskolin, Betulinsäure, Extrakte aus Plantago Arten, TGF-beta, Extrakte aus Ginkgo biloba, Glutamin und Glycolsäure Einsatz als Collagensynthesestimulatoren. Über eine synergistisch verstärkte Steigerung der Collagensynthese durch Einsatz der erfindungsgemäßen ternären Mischungen wurde hingegen nicht berichtet.

Eine ebenfalls bedeutende Rolle bei der Hautalterung spielen Matrixmetalloproteinasen, eine Gruppe von Enzymen, die in der Lage sind, die Makromoleküle der extrazellulären Matrix (ECM) zu der auch die Collagene zählen, proteolytisch abzubauen. Letztendlich bedeutet dies, dass MMPs der de novo Collagensynthese genau entgegenwirken. So wurde festgestellt, dass in alter Haut gegenüber junger Haut der Gehalt an MMPs deutlich erhöht ist (J. H. Chung et al., J. Invest. Dermatol, 2001, 117, 1218-1224). MMPs besitzen eine breite, oftmals überlappende Substratspezifität,

und in Kombination sind sie fähig, sämtliche Proteinkomponenten der extrazellulären Matrix zu zerlegen. Bisher wurden etwa 20 MMPs identifiziert. Sie werden im allgemeinen als inaktive Pro-Enzyme (Pro-MMP) sezerniert. In der menschlichen Haut spielen v. a. MMP-1 (Collagenase-1), MMP-2 (Gelatinase A), MMP-9 (Gelatinase B) und MMP-3 eine wichtige Rolle. MMP-1 spaltet neben Collagen-1 und -3 auch Pro-MMP-2 und Pro-MMP-9 und bewirkt dadurch deren Aktivierung. MMP-2 und MMP-9 gehören zu den Elastin abbauenden Proteasen (A. Thibodeau, Cosmetics & Toiletries 2000, 115 (11), 75-82).

Auch bei der durch exogene Faktoren bedingten vorzeitigen Hautalterung spielen MMPs eine entscheidende Rolle. So konnte in lichtgealterter Haut gegenüber lichtgeschützt gealterter Haut ein nochmals erhöhter Spiegel an MMPs detektiert werden (J. H. Chung et al., J. Invest. Dermatol, 2001, 117, 1218-1224). Sowohl für UVA- und UVB- als auch für Infrarot-Strahlung wurde die Induktion von Matrixmetalloproteinasen nachgewiesen. Diese Induktion konnte sowohl *in vitro* an kultivierten humanen dermalen Fibroblasten als auch *in vivo* an UVbestrahlter menschlicher Haut beobachtet werden. Auch die Stimulation mit Tabakrauch führte in humanen dermalen Fibroblasten zu einer Aufregelung der Expression von MMP-1 und -3 (J. Krutmann, Hautarzt 2003, 54, 809-817).

Die Verwendung von MMP-1 inhibierenden Stoffen (z.B. Retinylpalmitat, Propylgallat, Precocene, 6-Hydroxy-7-methoxy-2,2-dimethyl-1(2H)-benzopyran, 3,4-Dihydro-6-hydroxy-7-methoxy-2,2-dimethyl-1(2H)-benzopyran) zur Vorbeugung gegen die sonnenlicht- und/oder wärmeinduzierte Alterung der menschlichen Haut ist bekannt und wurde z.B. in der WO 01/74320 beschrieben.

Ferner ist bekannt, dass Matrixmetalloproteinasen insbesondere auch bei krankhaften Veränderungen der Mundhöhle, z.B. des Parodonts bedeutsam sind. Parodontitis (auch "Parodontose" genannt) ist eine Entzündung des Parodonts, also des Zahnhalteapparates. Das Parodont umfasst verschiedene Gewebe: das Zahnfleisch-Epithel (Gingiva), das Bindegewebe der Gingiva, die Zahnwurzelhaut (Desmodont), den Wurzelzement und den umgebenden Alveolarknochen. Das Desmodont befindet sich zwischen Wurzeloberfläche und dem Alveolarknochen. Es ist ein zellreiches Bindegewebe, das den Zahn im knöchernen Zahnfach, der Alveole verankert. Der Desmodontalspalt wird zu 53-74% von collagenen und oxytalanen Faserbündeln eingenommen. Der in den Wurzelzement und in den Alveolarknochen eingelassene Teil der desmodontalen Fasern verankert den Zahn in der Alveole. Zu den klinischen Hauptmerkmalen einer Parodontitis zählen Zahnfleischentzündung verbunden mit Wundbildung, Attachmentverlust, Bildung von Parodontaltaschen und Alveolarknochenabbau.

Hauptursache für Parodontitis ist der Zahnbelag oder "Plaque". Dieser besteht aus bestimmten Bestandteilen des Speichels, Speiseresten und vor allem aus Bakterien und deren Abbauprodukten. Diese besondere Form einer Infektionskrankheit wird in den meisten Fällen durch *Porphyromonas gingivalis, Bacteroides forsythus* und *Actinobacillus actinomycetemcomitans* hervorgerufen. Die kontinuierliche Freisetzung bakterieller Toxine, insbesondere von Lipopolysacchariden, löst vermutlich die Ausschüttung pro-inflammatorischer Mediatoren, wie z. B. IL-1beta, TNF-alpha und PGE2 in betroffenen Geweben des Patienten aus. Diese Signalstoffe regen die Infiltration immunkompetenter Zellen in das besiedelte Gewebe an. Die Einwanderung neutrophiler Granulozyten und Makrophagen führt im folgenden dann zu einer Zahnfleischentzündung (Gingivitis) und zur Freisetzung pro-inflammatorischer Mediatoren wie beispielsweise IL-1 und IL-6. Diese wiederum aktivieren in Haut und Schleimhaut die Synthese Matrix-degradierender Metallo-Proteinasen (Matrixmetalloproteinasen, MMPs), welche die extrazelluläre Matrix des umgebenden Bindegewebes zerstören. Dadurch dringen Bakterien, die zunächst mit dem Gingivalsaum interagierten, tiefer in das darunterliegende Bindegewebe ein, setzen dort Entzündungsprozesse und die Synthese von MMPs fort und lösen letztendlich die Verbindung der obersten Schicht des Epitheliums mit der Zahnwurzel. Als Konsequenz formt sich eine Zahnfleischtasche. Die Reaktion des Körpers ist die Entzündung der Gingiva und des Parodontiums mit Schädigung des Alveolarknochens. Im Endstadium der Parodontitis droht den Betroffenen ein massiver Zahnverlust. Weiterhin damit einher gehen Infektionen der Mundhöhlen sowie Wundbildung.

Studien (T. Kuboto et al., Arch. Oral. Biol. 1996, 41, 253-262; A. L. Ejeil et al., J. Periodontol. 2003, 74, 188-195) zeigten, dass die Levels einer Reihe von Matrixmetalloproteinasen (MMP-1, -3, -8, -9 und -13) bei Patienten mit Zahnfleischentzündung gegenüber Patienten mit gesundem Zahnfleisch signifikant erhöht sind. Die Levelhöhe korreliert mit der Schwere der Gingivitis bzw. Parodontitis. Weiterhin nehmen Collagenfasern signifikant mit zunehmender Ausprägung der Zahnfleischentzündung ab. MMP-9 fungiert dabei offenbar als Marker in einem frühen Stadium der Parodontitis (A. L. Ejeil et al., J. Periodontol. 2003, 74, 188-195). Die Kompensation dieser Effekte hervorgerufen durch den gesteigerten Aufbau von Collagen mit Hilfe der synergistisch wirksamen ternären Mischungen ist daher ebenfalls vorteilhaft angezeigt

Auch bei der Entstehung von Karies und nichtkariesbedingten Zahnhartsubstanzverlusten wie beispielsweise Erosionen spielen MMPs eine wichtige Rolle. Die Zähne sind hauptsächlich aus einer knochenähnlichen Substanz, dem Dentin (Zahnbein) aufgebaut. Im Bereich der aus dem Zahnfleisch herausragenden Zahnkrone ist das Dentin von dem schützenden Zahnschmelz überzogen. Das Dentin besteht zu etwa 30% aus einer zellfreien Grundsubstanz, die vor allem Glykoproteine enthält. In diesen sind Collagenfasern sowie anorganische Bestandteile eingelagert.

Die Entstehung von Karies und Erosionen geht einher mit der Demineralisierung der Zähne. Die Mineralstoffe sind maßgeblich für die Härte des Zahns verantwortlich. Durch die Bildung von Säuren durch orale Bakterien nach Verzehr zuckerhaltiger Lebensmittel einerseits, aber auch durch häufigen Kontakt mit stark säurehaltigen Getränken

(z. B. Fruchtsäften) und stark säurehaltiger Nahrung (Südfrüchte, Ananas etc.) kommt es zur Demineralisierung des Zahnschmelzes und bei weiterem Fortschreiten auch des Dentins. Das demineralisierte Dentin ist anfällig für Degradierung. In vitro konnte gezeigt werden, dass der Abbau der organischen Matrix notwendig für die Bildung eines Lochs im Zahn ist. Tjäderhane et al. (J. Dent. Res. 1998, 77, 1622-1629) detektierten MMP-2, MMP-8 und MMP-9 in Kariesläsionen und stellten fest, dass diese durch Säuren aktiviert werden. Die Verlangsamung und idealerweise die komplette Unterbindung dieser maßgeblich durch Collagenabbau hervorgerufen Effekte mit Hilfe der synergistisch wirksamen ternären Mischungen ist daher ebenfalls vorteilhaft angezeigt.

Pashley et al. (J. Dent. Res. 2004, 83, 216-221) zeigten, dass auch in Abwesenheit von Bakterien Degradierung von Collagenfasern der organischen Matrix in durch Säure teilweise demineralisiertem Dentin durch collagenolytisch aktive Proteasen stattfindet. Durch Zusatz von Chlorhexidin bzw. Protease-Inhibitoren (MMP-Inhibitor: Benzamidin Hydroclorid, Cysteinproteinase-Inhibitor: N-Ethylmaleimid, epsilon-Amino-n-Capronsäure, Serinprotease-Inhibitor: Phenylmethylsulfonylfluorid) wurde der Abbau der Collagenfasern verhindert. Die Verlangsamung und idealerweise die komplette Unterbindung dieser maßgeblich durch Collagenabbau hervorgerufen Effekte mit Hilfe der synergistisch wirksamen ternären Mischungen ist daher ebenfalls vorteilhaft angezeigt.

Die Gesunderhaltung bzw. Verlangsamung des Abbaus des Bindegewebes des Parodonts und der Collagenfasern der Zähne, einerseits durch Verhinderung von Schädigungen durch MMPs durch Einsatz von Matrixmetalloproteinase-Inhibitoren und andererseits durch den erhöhten Aufbau von Collagen durch Collagensynthese steigernde Wirkstoffe sind daher wichtige Strategien bei der Entwicklung neuer Wirkstoffe für den Bereich der Mundpflege bzw. der Mundhygiene. Um die beschriebenen Prozesse effektiv aufzuhalten, muss bereits zu einem sehr frühen Zeitpunkt die Schädigung durch MMPs und hier insbesondere durch MMP-1 (Collagenase-1), MMP-2 (Gelatinase A), MMP-8 (Collagenase-2) und MMP-9 (Gelatinase B) inhibiert werden.

In mehreren Publikationen wird die Verwendung synthetischer MMP- Inhibitoren bei parodontalen Erkrankungen beschrieben (M. E. Ryan et al., Curr. Opin. Periodontal. 1996, 3, 85-96; R. Gendron et al., Clin. Diagn. Lab. Immunol. 1999, 6, 437-439). Der Ausgleich dieser Effekte durch gesteigerten Aufbau von Collagen mit Hilfe der synergistisch wirksamen ternären Mischungen ist ebenfalls vorteilhaft angezeigt, da Stoffe, die die Collagensynthese steigern dem negativen Effekte des MMP-induzierten Collagenabbaus durch de novo Synthese entgegenwirken.

Es war daher die Aufgabe der vorliegenden Erfindung, Mittel anzugeben, die die de novo Collagensynthese gegenüber dem Stand der Technik signifikant steigern und somit zum Verlangsamen der Hautalterung und zum Schützen der Haut und Schleimhaut des Parodonts und zum Schützen der organischen Matrix des Dentins beitragen bzw. einer Schädigung und/oder einem übermäßigen Abbau des Collagen entgegenwirken. Die erfindungsgemäßen Mittel sollten, soweit eine Anwendung für die Haut eines Benutzers angestrebt wird, nach Möglichkeit besonders gegen die extrinsische Hautalterung und die damit in Verbindung gebrachten exogenen Einflussfaktoren wirksam sein. Die anzugebenden Mittel sollten nach Möglichkeit natürlichen Ursprungs sein, leicht herstellbar, gut lagerfähig und in zahlreichen unterschiedlichen Zubereitungen, insbesondere in kosmetischen und pharmazeutischen Zubereitungen sowie in Zubereitungen zur Mundhygiene, einsetzbar sein. Darüber hinaus sollten Herstellverfahren für entsprechende Mittel und deren Verwendungen angegeben werden.

Dabei wird unter einem Mundhygieneprodukt (nachfolgend auch genannt Mundpflegeprodukt oder mundhygienische Zubereitung) im Sinne der Erfindung eine der dem Fachmann geläufigen Formulierungen zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind Cremes, Gele, Pasten, Schäume, Emulsionen, Suspensionen, Areosole, Sprays als auch Kapseln, Granulate, Pastillen, Tabletten, Bonbons oder Kaugummis, ohne dass diese Aufzählung für die Zwecke dieser Erfindung limitierend verstanden werden soll.

Erfindungsgemäß wird deshalb eine tenäre Mischung angegeben enthaltend, bestehend im wesentlichen aus oder bestehend aus

a) einem Extrakt aus Aloe-Pflanzen-Material,

b) Ascorbinsäure und/oder deren Derivat(e), vorzugsweise zumindest einem Solvat und/oder Salz der Ascorbinsäure und/oder zumindest einem Ascorbinsäure-Prodrug, und

c) einem Extrakt aus Rosaceae-Pflanzen-Material,

ausgenommen einem After-sun-Balsam ("after-sun balm") enthaltend gleichzeitig 3.0 Gew.-% an Aloe barbadensis-Blattextrakt 10/1 in Wasser, 1.0 Gew.-% Natrium-ascorbylphosphat und 0.5 Gew.-% Maltodextrin-Rubus fruticosus-Blattextrakt, jeweils bezogen auf den gesamten Balsam.

Kosmetische und pharmazeutische Mittel, die die Collagensynthese steigern, sind als solche bereits bekannt. Bereits häufig für diesen Zweck zum Einsatz gelangende Mittel sind Retinolderivate wie Retinsäure, Retinal, Retinol,

Retinylacetat oder Retinylpalmitat, Ascorbinsäure sowie deren Derivate wie insbesondere Ascorbylpalmitat, Magnesiumascorbylphosphat, Natriumascorbylphosphat sowie Ascorbyl-alpha- und beta-Gluosid. Daneben werden eine ganze Reihe weiterer Wirkstoffe sowie Pflanzenextrakten als Stimulatoren der Collagensynthese beschrieben: peptidische Stoffe und deren Derivate wie z.B. Carnitin, Carnosin, Kreatin, Matrikine Peptide (e.g. lysyl-threonyl-threonyl-lysyl-serine) sowie weitere peptidische Strukturen wie palmitoylierte Pentapeptide (z.B. Matrixyl/Fa. Sederma) oder das Oligopeptid mit dem Handelsnamen Vincipeptide (Fa. Vincience/Frankreich), sowie Substanzen wie Asiatsäure (Asiatic acid), Madecassic Säure, Madecassosid, Asiaticosid, Extrakte aus Aloe- und Centella-Arten, Niacinamid, Astaxanthin, Glucane z.B aus Hefen und Hafer, Sojaextrakte sowie Sojaisoflavone wie Genistein und Daidzein sowie weiterhin Rutin, Chrysin, Morin, Betelnuß Alkaloide, Forskolin, Betulinsäure, Extrakte aus Plantago Arten, TGF-beta, Extrakte aus Ginkgo biloba, Glutamin und Glycolsäure. In den meisten Fällen ist über den biologischen Mechanismus der Collagensynthese steigernden Wirkung jedoch nur wenig bekannt.

Über die Verwendung ternärer Mischungen enthaltend Aloe Extrakt, mindestens einen Stoff ausgewählt aus der Gruppe Ascorbinsäure und deren Derivate sowie mindestens einen Rosaceen-Extrakten, vorzugsweise Himbeerblattextrakt, Erdbeerblattextrakt, Brombeerblattextrakt oder Mädesüßkrautextrat zur Steigerung der Collagensynthese wurde hingegen bis dato nicht in der Literatur berichtet.

Überraschenderweise hat sich in umfangreichen eigenen Untersuchungen gezeigt, dass insbesondere Mischungen enthaltend Aloe Extrakt, mindestens einen Stoff ausgewählt aus der Gruppe Ascorbinsäure und deren Derivate sowie mindestens einen Rosaceen-Extrakten, vorzugsweise Himbeerblattextrakt, Erdbeerblattextrakt, Brombeerblattextrakt oder Mädesüßkrautextrakt hervorragende Collagensynthese steigernde Eigenschaften besitzen. Die Wirksamkeit war hierbei gegenüber Produkten bestehend ausschließlich aus Aloe Extrakt, ausschließlich aus mindestens einem Stoff ausgewählt aus der Gruppe Ascorbinsäure und deren Derivate oder ausschließlich aus mindestens einem Rosaceen-Extrakten, vorzugsweise Himbeerblattextrakt, Erdbeerblattextrakt, Brombeerblattextrakt oder Mädesüßkrautextrakt synergistisch verstärkt. Die synergistisch verstärkte Wirksamkeit der ternären Mischungen konnte durch Berechnung von Synergieindices (SI-Werten) mit Hilfe der Kull'schen Gleichung (Literatur: F.C.Kull et al, Applied Microbiology Vol.9, p. 538-541 (1961); D.C.Steinberg, Cosmetics & Toiletries Vol.115(11), 59-62 (2000)) eindeutig belegt werden.

Ein weiterer, unerwarteter Vorteil der erfindungsgemäßen Mischungen ist ihr geringer Eigengeruch, wodurch sie sich besonders vorteilhaft in der Kosmetik und dort vor allem im Bereich der leave-on Produkte einsetzen lassen, da die erfindungsgemäßen Mischungen somit kaum oder gar nicht zu einer Veränderung des Aussehens und Geruchs einer den Extrakt auch in hohen Konzentrationen enthaltenden kosmetischen Zubereitung führt. Zudem besitzen die Mischungen einen schwachen, angenehmen Eigengeschmack. Dies macht die Mischungen zusätzlich geeignet zur Verwendung in Mundhygieneprodukten, da bei einer Verwendung im Mundraum der Geschmack ein wichtiges Akzeptanzkriterium für Benutzer ist und damit letztlich auch über den Erfolg einer Anwendung des Mundhygieneproduktes entscheidet.

Der Extrakt aus Aloe-Pflanzen-Material ist besonders bevorzugt hergestellt aus Aloe barbadensis und Aloe vera.

Bevorzugte Ascorbinsäure-Derivate sind: Natrium Ascorbylphosphat, Magnesium Ascorbylphosphat, 3-O-Ethyl Ascorbinsäure, Allantoin Ascorbat, Aminopropyl Ascorbyl Phosphat, Ascorbylpalmitat, Araboascorbinsäure Mononatriumsalz, Ascorbinsäure Polypeptid, Ascorbosilane C, Ascorbyl Dipalmitat, Ascorbyl-alpha-Glucosid, Ascorbyl-beta-Glucosid, Ascorbyl Inositol Nikotinat, Ascorbyl Linoleat, Ascorbyl Methylsilanol Pectinat, Ascorbyl Nikotinamid, Ascorbyl Stearat, Ascorbyl Tetraisopalmitat, Ascorbyl Tocopheryl Maleat, Calcium Ascorbat, Chitosan Ascorbat, D-Arabino-Ascorbinsäure, Dinatrium Ascorbyl Sulfate, Glucosamine Ascorbat, Inositol Hexanikotinat Hexaascorbat, Isoascorbinsäure, L-Ascorbinsäure, 2-(Dihydrogen Phosphat), 2-[3-Cholest-5-en-3-yl Hydrogen Phosphat], 2-O--D-Glucopyranosyl-, L-Ascorbinsäure, 3-O-Ethyl Ether, Magnesium Ascorbat, Magnesium Ascorbylborat, Methoxy PEG-7 Ascorbinsäure, Methylsilanol Ascorbat, Kalium Ascorbyl Tocopheryl Phosphat, Kalium Ascorbylborate, Natrium Ascorbat, Natrium Ascorbyl/ Cholesteryl Phosphat, Natrium IsoAscorbat, Natrium L-Ascorbyl-2-Phosphat, Tetrahexyldecyl Ascorbat,Rosaceen-Extrakte werden dabei vorzugsweise erhalten mit einem Extraktionsmittel bestehend aus reinem Wasser, reinem Ethanol oder aus Ethanol-Wasser-Gemischen in beliebigen Mengenverhältnissen von 99:1 (99 Gew.-Teile Ethanol gemischt mit 1 Gew.-Teilen Wasser) bis 1:99, (1 Gew.-Teil Ethanol gemischt mit 99 Gew.-Teilen Wasser). Die Extrakte werden vorzugsweise durch gängige, schonende Trocknungsverfahren wie z.B. durch Sprühtrocknung oder Vakuumbandtrocknung zu pulverförmigen Produkten weiterverarbeitet. Der Zusatz von Trägerstoffen wie z.B. Maltodextrin kann hierbei im Konzentrationsbereich von 10% bis 95% und vorzugsweise im Konzentrationsbereich von 30% bis 80% liegen.

Alle genannten Wirkstoffe können entweder kristallin oder in Pulverform hergestellt werden. Bevorzugte Verfahren zur Herstellung der erfindungsgemäßen ternären Mischungen sind daher alle in der Kosmetikindustrie und in der pharmazeutischen Industrie gängigen Verfahren zur Herstellung stabiler und homogen verteilter Pulvermischungen. Darüber hinaus kann es aber auch vorteilhaft sein, die erfindungsgemäßen ternären Mischungen in Form von Lösungen herzustellen. Besonders geeignete Lösungsmittel sind hierbei Wasser, einwertige Alkohole einer C-Zahl von 1 bis 5 wie insbesondere Ethanol, Propanol und Butanol sowie mehrfach hydroxylierte aliphatische Verbindungen wie insbesondere Glycerin, Proylenglycol, Butylenglycol sowie 1,2-Alkandiole mit einer C-Zahl von 3-10.

Die Konzentrationsverhältnisse der erfindungsgemäßen ternären Mischungen enthaltend mindestens jeweils

eine Komponente a) bis c) können dabei beliebig folgendermaßen gewählt werden, wobei folgende Mengenverteilungen erfindungsgemäß bevorzugt sind:

Komponente a): 0,01 Gewichtsprozent bis 99 Gewichtsprozent und/oder

Komponente b): 0,01 Gewichtsprozent bis 99 Gewichtsprozent und/oder

Komponente c): 0,01 Gewichtsprozent bis 99 Gewichtsprozent, jeweils bezogen auf die Summe der Komponenten a), b) und c).

Bevorzugte synergistisch wirksame ternäre Mischungen enthalten hingegen einen Überschuss an Komponente a), eine mittlere Menge an Komponente b), und eine gegenüber den Komponenten a) und b) geringe Menge an Komponente c). Bevorzugt sind daher insbesondere Mischungen, bei denen die Anteile an

Komponente a): 33 -99 Gew.-%

Komponente b): 0,99 - 32,99 Gew.-%

Komponente c): 0,01 - 10 Gew.-% betragen, jeweils bezogen auf die Summe der Komponenten a), b) und c).

Mit diesen Mengenverhältnissen kann insbesondere eine synergistisch gesteigerte Collagen-Synthese bewirkt werden.

Besonders bevorzugte synergistische wirksame ternäre Mischungen enthalten die Komponenten a) bis c) in folgender Mengenverteilung:

Komponente a): 80 bis 98 Gew.-%

Komponente b): 1,9 bis 15 Gew.-%

Komponente c): 0,1 bis 4 Gew.-%

Eine ganz besonders bevorzugte, synergistische wirksame ternäre Mischung enthält die Komponenten a) bis c) in folgender Mengenverteilung:

Komponente a): 96,51 Gew.-%

Komponente b): 3,22 Gew.-%

Komponente c): 0,27 Gew.-%, jeweils bezogen auf die Summe der Komponenten a), b) und c).

Als Himbeerblatt-Extrakt enthaltende, synergistisch wirksame ternäre Mischung ist folgende Zusammensetzung besonders bevorzugt:

Komponente a): 85-92 Gew.-%, vorzugsweise 88-92 Gew.-%, besonders bevorzugt 90 Gew.-%,

Komponente b): 7-10 Gew.-%, vorzugsweise 9 Gew.-%,

Komponente c): 0,5-2 Gew.-%, vorzugsweise 0,8-1,2 Gew.-%, besonders bevorzugt 1 Gew.-%, jeweils bezogen auf die Summe der Komponenten a), b) und c), wobei Komponente b) aus Magnesium-Ascorbylphosphat und Komponente c) aus Himbeerblatt-Extrakt besteht.

Eine in diesem Zusammenhang besonders bevorzugte, synergistische wirksame ternäre Mischung enthält die Komponenten a) bis c) in folgender Mengenverteilung:

Komponente a): 90,00 Gew.-%

Komponente b), nämlich Magnesium-Ascorbylphosphat: 9,00 Gew.-%

Komponente c), nämlich Himbeerblatt-Extrakt: 1,00 Gew.-%, jeweils bezogen auf die Summe der Komponenten a),

b) und c).

Die Konzentration der ternären Mischungen (in flüssiger und/oder aufkonzentrierter Form) in (insbesondere topisch zu applizierenden) kosmetischen, mundhygienischen und/oder pharmazeutischen Zubereitungen liegt vorzugsweise im Bereich von 0,001 bis 20 Gew.-%, bevorzugt im Bereich von 0,01 bis 10 Gew.-% und besonderes bevorzugt im Bereich von 0,1 bis 5 Gew.-%, bezogen auf die gesamte Zubereitung. Diese Konzentrationen sind insbesondere bevorzugt zur synergistischen Stimulation der Collagensynthese. Sie sind ferner bevorzugt

- zum Verlangsamen der Hautalterung und/oder

- zum Vorbeugen und/oder Verlangsamen von Parodontitis und/oder Karies.

Die erfindungsgemäßen Mischungen werden erfindungsgemäß bevorzugt hergestellt durch ein Verfahren umfassend das Mischen der Komponenten a), b) und c) in ihren jeweils gewählten Mengen zum Erhalten einer homogenen pulverförmigen Mischung.

Die erfindungsgemäßen ternären, in Pulverform oder als Flüssigkeit vorliegenden Mischungen können vorzugsweise weiterverarbeitet werden zu einem finalen, ebenfalls erfindungsgemäßen Endprodukt in vorzugsweise flüssiger Form, indem das erfindungsgemäße Herstellverfahren ergänzt wird um den Schritt: Versetzen der ternären Mischung mit einem weiteren pharmazeutisch, mundhygienisch und/oder kosmetisch akzeptablen Lösungsmittel oder Trägerstoff.

Die erfindungsgemäßen, die Collagensynthese steigernden ternären Mischungen eignen sich überraschend in hervorragender Weise als kosmetisches und/oder pharmazeutisches Mittel zur Vorbeugung, Linderung und Behandlung unerwünschter Veränderungen in der Mundhöhle, die auf eine erhöhte Aktivität von MMPs und dem damit einhergehenden Abbau von Collagen zurückzuführen sind, da sie die negativen Effekte der MMPs durch gesteigerte de novo Synthese von Collagen kompensieren. Eine kombinierte Verwendung der erfindungsgemäßen, die Collagensynthese steigernden Mischungen gemeinsam mit nicht erfindungsgemäßen MMP-Inhibitoren kann hier ebenfalls vorteilhaft sein.

Pharmazeutisch, mundhygienisch bzw. kosmetisch akzeptabel ist im Sinne dieser Erfindung ein solcher Trägerstoff, der für den Organismus, an dem er verwendet werden soll, zumindest nicht toxisch ist. Bevorzugte kosmetisch, mundhygienisch oder pharmazeutisch akzeptable Feststoffe sind pulverförmiges Maltodextrin, Lactose, Siliciumdioxid oder Glucose sowie Mischungen zweier oder mehrerer dieser Feststoffe. Ein für mundhygienische Zwecke erfindungsgemäß besonders bevorzugter akzeptabler Feststoff ist Siliciumdioxid. Weitere erfindungsgemäß für mundhygienische Zwecke verwendbare akzeptable Feststoffe sind Hydrokolloide wie Stärken, abgebaute Stärken, chemisch oder physikalisch modifizierte Stärken, modifizierte Cellulosen, Gummi Arabicum, Ghatti-Gum, Traganth, Karaya, Carrageenan, Pullulan, Curdlan, Xanthan Gum, Gellan Gum, Guarkernmehl, Johannisbrotkernmehl, Alginate, Agar, Pektin und Inulin sowie Mischungen zweier oder mehrerer dieser Feststoffe, insbesondere auch mit Siliciumdioxid.

Darüber hinaus kann die feste oder flüssige ternäre Mischung auch erfindungsgemäß zu einer vorzugsweise flüssigen Zubereitung weiterverarbeitet werden, indem die ternäre Mischung mit einem weiteren Lösungsmittel ausgewählt aus der Gruppe bestehend aus Glycerin, 1,2-Propylenglycol, 1,3-Butylenglycol, Ethanol, Wasser und Mischungen zweier oder mehrerer der genannten Lösungsmittel mit Wasser. Solche erfindungsgemäß hergestellten Endprodukte sind insbesondere für kosmetische und mundhygienische Zwecke gut weiterverarbeitbar. Gegebenenfalls können diese erfindungsgemäßen Zubereitungen hergestellt werden unter Zusatz eines Konservierungsmittels, Lösungsvermittlers oder Antioxidans.

Weiterhin können die erfindungsgemäßen festen oder flüssigen ternären Mischungen oder die eine erfindungsgemäße feste oder flüssige ternäre Mischung enthaltende flüssige oder feste Zubereitung auch erfindungsgemäß durch Verkapselung weiterverarbeitet werden. Erfindungsgemäß werden die erfindungsgemäßen festen oder flüssigen ternären Mischungen oder die eine erfindungsgemäße feste oder flüssige ternäre Mischung enthaltende flüssige oder feste Zubereitung verkapselt mit einem festen Hüllmaterial, das vorzugsweise ausgewählt ist aus Stärken, abgebaute oder chemisch oder physikalisch modifizierte Stärken (insbesondere Dextrine und Maltodextrine), Gelatinen, Gummi Arabicum, Agar-Agar, Ghatti-Gummi, Gellan Gum, modifizierte und nicht modifizierte Cellulosen, Pullulan, Curdlan, Carrageenane, Alginsäure, Alginate, Pektin, Inulin, Xanthan Gum und Mischungen zweier oder mehrerer der genannten Substanzen.

Vorzugsweise wird das feste Hüllmaterial gewählt aus Gelatine (vorteilhaft sind Schweine-, Rinder-, Hühner- und/oder Fischgelatinen und Mischungen davon, vorzugsweise umfassend mindestens eine Gelatine mit einem Bloom-Wert von größer oder gleich 200, bevorzugt mit einem Bloom-Wert von größer oder gleich 240), Maltodextrin (vorzugsweise aus Mais, Weizen, Tapioka oder Kartoffel gewonnen, bevorzugte Maltodextrine weisen einen DE-Wert Im Bereich von 10 - 20 auf), modifizierte Cellulose (z.B. Celluloseether), Alginate (z.B. Na-Alginat), Carrageenan (beta-, iota-, lambda- und/oder kappa- Carrageenan), Gummi Arabicum, Curdlan und/oder Agar-Agar. Gelatine wird insbesondere wegen ihrer guten Verfügbarkeit in unterschiedlichen Bloom-Werten bevorzugt verwendet. Besonders bevorzugt namentlich für mundhygienische Zwecke sind nahtlose Gelatine- oder Alginatkapseln, deren Hülle sich im Mund sehr schnell auflöst bzw. beim Kauen zerplatzt und so den Wirkstoff in der Mundhöhle freisetzen. Die Herstellung kann beispielsweise erfolgen wie beschrieben in EP 0 389 700, JP 7 196 478, US 4,251,195, US 6,214,376 WO 03/055587 oder WO 2004/050069.

Diesynergistisch wirksame ternäre Mischung enthaltend mindestens einen Extrakt aus einer Aloe Art ausgewählt aus a), mindestens einen Stoff aus der Gruppe Ascorbinsäure und deren Derivate ausgewählt aus b) sowie mindestens einen Rosaceen-Extrakten, vorzugsweise Himbeerblattextrakt, Erdbeerblattextrakt, Brombeerblattextrakt oder Mädesüßkrautextrakt ausgewählt aus c) lässt sich vorteilhaft überall dort in der Kosmetik einsetzen, wo mit einer Steigerung der Collagensynthese kosmetisch erwünschte Effekte verbunden sind. Vorzugsweise wird die Mischung jedoch als Wirkstoff gegen die natürliche und gegen die vorzeitige, beispielsweise Sonnenlicht-induzierte Hautalterung und gegen Falten eingesetzt. Dazu wird er vorzugsweise topisch auf die zu behandelnde Haut appliziert.

Wesentliche Anwendungsgebiete sind hierbei kosmetische, insbesondere dermatologische Zubereitungen, die (abgesehen von den erfindungsgemäßen Komponenten a) - c) ) wie üblich zusammengesetzt sind und dem kosmetischen, insbesondere dermatologischen Lichtschutz, zur Behandlung, der Pflege und der Reinigung der Haut und/oder der Haare oder als Schminkprodukt in der dekorativen Kosmetik dienen. Entsprechend können derartige Zubereitungen, je nach ihrem Aufbau beispielsweise als Hautschutzcreme, Tages- oder Nachtcreme, Augencreme, Sonnenschutz- oder After-sun-Lotion, Nährcreme, Pflegemaske, Gel-Pads, Gesichtswasser, feuchte Pflege- und Reinigungstücher, Reinigungsmilch, Reinigungsseife, Schaum- oder Duschbad, Deodorant, Antitranspirant, Haarshampoo, Haarpflegemittel, Haarconditioner, Haarcoloration, Haarstylingmittel verwendet werden und dabei bevorzugt als Emulsion, Lotion, Milch, Fluid, Creme, Hydrodispersionsgel, Balm, Spray, alkoholische oder wässrig/alkoholische Lösung, Schaum, Puder, Flüssigseife, Seifenstück, Shampoo, Roll-on, Stick oder Make-up vorliegen. In Haarbehandlungsmitteln richtet sich die Verwendung vorzugsweise auf den Haarboden oder die Kopfhaut.

Des weiteren wurde gefunden, dass eine erfindungsgemäße synergistisch wirksame ternäre Mischung in einer zur Stimulation der Collagensynthese ausreichenden Konzentration zur Behandlung von Paradontitis und Karies wirkt und diesen insbesondere vorbeugt und ihre Ausprägung verzögert. Daher wird erfindungsgemäß gelehrt, erfindungsgemäße synergistisch wirksame ternäre Mischungen und entsprechende Mundpflegeprodukte vorteilhaft überall dort in der Mundpflege bzw. Mundhygiene einzusetzen, wo mit einer Collagensynthese-Steigerung erwünschte Effekte verbunden sind. Vorzugsweise wird die synergistisch wirksame ternäre Mischung bzw. die kosmetische, pharmazeutische oder Mundpflegezubereitung jedoch als Wirkstoff zur Vorbeugung, Verlangsamung und Heilung von Paradontitis und Karies, zur Behandlung von Infektionen der Mundhöhle sowie zur Wundheilung in der Mundhöhle eingesetzt. Dazu wird die erfindungsgemäße ternäre Mischung vorzugsweise äußerlich mit der Mundschleimhaut und den Zähnen in Kontakt gebracht. Solche Mischungen und mundpflegende Formulierungen können insbesondere erfindungsgemäß dazu verwendet werden, die Zahnsubstanz und die Mundhöhle zu reinigen sowie infektiös bedingte Entzündungen und Wunden in der Mundhöhle zu heilen.

Die Konzentration der ternären Mischungen (in flüssiger und/oder aufkonzentrierter Form) in (insbesondere topisch zu applizierenden) kosmetischen, mundhygienischen und/oder pharmazeutischen Zubereitungen liegt vorzugsweise im Bereich von 0,001 bis 20 Gew.-%, bevorzugt im Bereich von 0,01 bis 10 Gew.-% und besonderes bevorzugt im Bereich von 0,1 bis 5 Gew.-%, bezogen auf die gesamte Zubereitung. Die erfindungsgemäßen Mischungen lassen sich ohne Schwierigkeiten in gängige kosmetische oder dermatologische Formulierungen wie Pumpsprays, Aerosolsprays, Cremes, Salben, Tinkturen, Lotionen, Nagelpflegeprodukte und dergleichen einarbeiten. Hierbei ist es auch möglich und in manchen Fällen vorteilhaft, einen erfindungsgemäß verwendeten Brombeerblatt-Extrakt mit weiteren Wirkstoffen zu kombinieren, beispielsweise mit anderen Wirkstoffen gegen Hautalterung und Falten. Diese Brombeerblatt-Extrakt enthaltenden kosmetischen und/oder dermatologischen Formulierungen können hierbei ansonsten wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Erfindungsgemäße Zubereitungen, die erfindungsgemäße ternäre Mischungen enthalten, können auch weitere Collagensynthese steigernde Wirkstoffe und Wirkstoffkombinationen insbesondere gegen Hautalterung und Falten oder für den Mundpflegebereich (insbesondere gegen Parodontitis und Karies, gegen Mundschleimhautentzündungen und zur Wundheilung enthalten. Erfindungsgemäß können hierbei alle für die Mundhygiene, für kosmetische und/oder dermatologische Anwendungen geeigneten oder gebräuchlichen Wirkstoffe verwendet werden. Besonders bevorzugt sind dabei peptidische Stoffe und deren Derivate wie z.B. Carnitin, Carnosin, Kreatin, Matrikine Peptide (e.g. lysyl-threonyl-threonyl-lysyl-serine) sowie weitere peptidische Strukturen wie palmitoylierte Pentapeptide (z.B. Matrixyl/Fa. Sederma) oder das Oligopeptid mit dem Handelsnamen Vincipeptide (Fa. Vincience/Frankreich), Substanzen wie Asiatsäure (Asiatic acid), Madecassic Säure, Madecassosid, Asiaticosid, Extrakte aus Centella-Arten, Niacinamid, Astaxanthin, Glucane z.B aus Hefen und Hafer, Sojaextrakte sowie Sojaisoflavone wie Genistein und Daidzein, weiterhin Rutin, Chrysin, Morin, Betelnuß-Alkaloide, Forskolin, Betulinsäure, Extrakte aus Plantago Arten, Extrakte aus Ginkgo biloba, Catechin (Catechine (z.B. Epigallocatechingallat)-reiche Extrakte aus grünem oder schwarzem Tee, Polyphenole (oligomere Procyanidine) aus Wein, Retinsäure, Retinal, Retinol, Retinolpalmitat sowie weitere Abkömmlinge des Retinol, Glutamin, Glycolsäure und TGF-beta.

Im Anwendungsbereich der Mundhygiene können die erfindungsgemäßen ternären Mischungen in die verschiedensten Darreichungsformen eingearbeitet werden, ohne auf eine oder wenige spezielle Darreichungsformen festgelegt zu sein, da sie vorteilhafterweise mit einer großen Vielzahl an üblichen Hilfs- und Zusatzstoffen harmoniert. Es ist dabei vorteilhaft, die erfindungsgemäßen Mundhygieneprodukte abzupuffern. Besonders vorteilhaft und bevorzugt ist ein pH-Bereich von 3,5 bis 10,0. Bevorzugte Mundhygieneprodukte sind vorzugsweise Zahncremes, Zahnpasten, Zahngele, Mundwässer, Mundspülungen, Flüssigkeiten zum Gurgeln und Mund- oder Rachensprays sowie Lutschpastillen, Lutschtabletten, Bonbons, Kaugummis, Kaubonbons und zahnpflegende Kaugummis.

Es ist auch für Mundhygienezwecke möglich und meist vorteilhaft, eine erfindungsgemäße ternäre Mischung mit anderen Einsatzstoffen zu kombinieren, dabei insbesondere mit MMP-Inhibitoren, beispielsweise aber auch mit antimikrobiell wirksamen oder entzündungshemmenden Stoffen, Aromastoffen, Geschmackstoffen und/oder Hilfsstoffen.

Bevorzugt in Kombination zu verwendende MMP-Inhibitoren sind hierbei Retinylpalmitat, Propylgallat, Precocene, 6-Hydroxy-7-methoxy-2,2-dimethyl-1(2H)-benzopyran, 3,4-Dihydro-6-hydroxy-7-methoxy-2,2-dimethyl-1(2H)-benzopyran, Benzamidin Hydroclorid, die Cysteinproteinase-Inhibitoren N-Ethylmaleimid und epsilon-Amino-n-Capronsäure der Serinprotease-Inhibitor: Phenylmethylsulfonylfluorid, Collhibin (Fa. Pentapharm; INCI: Hydrolyzed Rice Protein) Oenotherol (Fa. Soliance; INCI: Propylene Glycol, Aqua, Oenothera biennis root extract Ellagsäure und Ellagitannine, z.B. aus Granatapfel), Phosphoramidon Hinokitiol, EDTA, Galardin, EquiStat (Fa. Collaborative Group; Apfelfruchtextrakt, Sojasamenextrakt: Ursolsäure, Sojaisoflavone & Sojaproteine), Salbeiextrakte, MDI (Fa. Atrium; INCI: Glycosaminoglycans), Fermiskin (Fa. Silab/Mawi; INCI: Water and) Lentinus Edodes Extract), Actimp 1.9.3. (Fa. Expanscience/ Rahn; INCI: Hydrolyzed Lupine Protein), Lipobelle Soyaglycone (Fa. Mibelle; INCI: Alcohol, Polysorbate 80, Lecithin and Soy Isoflavones), Extrakte aus grünem und schwarzem Tee sowie zahlreiche weitere Pflanzenextrakte die in W0 02/069992 (siehe Tabellen 1-12) gelistet sind.

Die erfindungsgemäßen Mundhygieneprodukte können im weiteren Hilfsstoffe enthalten, wie sie üblicherweise in solchen Zubereitungen verwendet werden, z.B. Konservierungsmittel, Abrasiva, antibakterielle Mittel, entzündungshemmende Mittel, irritationsverhindernde Mittel, irritationshemmende Mittel, antimikrobielle Mittel, Antioxidantien, Adstringentien, antiseptische Mittel, Antistatika, Binder, Puffer, Trägermaterialien, Chelatbildner, Zellstimulantien, reinigende Mittel, pflegende Mittel, oberflächenaktive Substanzen, deodorierende Mittel, Weichmacher, Bakterizide, Emulgatoren, Enzyme, ätherische Öle, Filmbildner, Fixateure, Schaumbildner, Schaumstabilisatoren, Substanzen zum Verhindern des Schäumens, Schaumbooster, gelierende Mittel, gelbildende Mittel, feuchtigkeitsspendende Mittel, anfeuchtende Substanzen, feuchthaltende Substanzen, bleichende Mittel, optisch aufhellende Mittel, schmutzabweisende Mittel, reibungsverringernde Mittel, Gleitmittel, Trübungsmittel, deckfähige Mittel, Glanzmittel, Polymere, Pulver, Proteine, abschleifende Mittel, Silicone, hautberuhigende Mittel, hautreinigende Mittel, hautpflegende Mittel, hautheilende Mittel, kühlende Mittel, hautkühlende Mittel, wärmende Mittel, hautwärmende Mittel, Stabilisatoren, suspendierende Mittel, Verdickungsmittel, Vitamine, Öle, Wachse, Fette, Phospholipide, gesättigte Fettsäuren, ein- oder mehrfach ungesättigte Fettsäuren, $\alpha$-Hydroxysäuren, Polyhydroxyfettsäuren, Verflüssiger, Farbstoffe, farbschützende Mittel, Pigmente, Aromen, Geschmackstoffe, Riechstoffe oder andere übliche Bestandteile einer mundhygienischen Formulierung wie Alkohole, Polyole, Elektrolyte, organische Lösungsmittel, Süßstoffe, Zuckeraustauschstoffe, Kieselsäuren, Calciumkarbonat, Calciumhydrogenphosphat, Aluminiumoxid, Fluoride, Zink, Zinn-, Kalium-, Natrium- und Strontiumsalze, Pyrophosphate, Wasserstoffperoxid, Hydroxyapatite.

Sofern das Mundhygieneprodukt eine Lösung oder Lotion ist, können beispielsweise die WO2005/123101 genannten Lösungsmittel verwendet werden. Selbstverständlich können auch Mischungen der vorstehend genannten Lösungsmittel verwendet werden.

Beispiele für Geschmackstoffe oder Aromen, die neben einer erfindungsgemäßen ternären Mischung Bestandteil eines erfindungsgemäßen Mundhygieneprodukts sein können, finden sich z.B. in K. Bauer, D. Garbe, H. Surburg, Common Fragrance and Flavor Materials, 4th. Ed., Wiley-VCH, Weinheim 2001 oder auch in S. Arctander, Perfume and Flavor Chemicals, Vol. I und II, Montclair, N. J., 1969, Selbstverlag.

Beispiele für natürliche Aromen, die neben einer erfindungsgemäßen ternären Mischung Bestandteil eines erfindungsgemäßen Mundhygieneprodukts sein können, sind in WO2005/123101 genannt.

Beispiele für einheitliche Aromastoffe, die neben einer erfindungsgemäßen ternären Mischung Bestandteil eines erfindungsgemäßen Mundhygieneprodukts sein können, sind ebenfalls in WO2005/123101 genannt.

Verbindungen mit physiologischem Kühleffekt (Kühlsubstanzen), die neben einer erfindungsgemäßen ternären Mischung Bestandteil eines erfindungsgemäßen Mundhygieneprodukts und/oder eines erfindungsgemäßen kosmetischen, dermatologischen und/oder pharmazeutischen Produkts sein können, sind in WO2005/123101 genannt. Erfindungsgemäß bevorzugt sind Mundhygieneprodukte, die neben I-Menthol mindestens eine, besonders bevorzugt mindestens zwei weitere Kühlsubstanzen enthalten.

Komponenten, die ein Wärme-, Schärfe-, Kribbel- oder Prickelgefühl auf der Haut oder auf den Schleimhäuten

hervorrufen, insbesondere Aromastoffe mit einem wärmeerzeugenden Effekt und/oder scharf schmeckende Verbindungen (Scharfstoffe), die neben einer erfindungsgemäßen ternären Mischung Bestandteil eines erfindungsgemäßen Mundhygieneprodukts und/oder kosmetischen, dermatologischen bzw. pharmazeutischen Produkts sein können sind ebenfalls in WO2005/123101 genannt.

Weitere Komponenten, die neben einer erfindungsgemäßen ternären Mischung Bestandteil eines erfindungsgemäßen Mundhygieneprodukts sein können, sind z. B. Stoffe zur Verbesserung der Mundhygiene, wie beispielsweise zahnpflegende und/oder erfrischende Stoffe. Zu den Stoffen zur Verbesserung der Mundhygiene zählen beispielsweise Stoffe zu Bekämpfung oder Verhinderung von Plaque, Zahnstein oder Karies sowie solche zur Bekämpfung oder Verhinderung von Mundgeruch. Es sei in diesem Zusammenhang auf die US 5,043,154 hingewiesen. Beispielhaft zu verwendende Stoffe sind im weiteren in WO2005/123101 genannt.

Ein erfindungsgemäßes Mundhygieneprodukt kann neben einer erfindungsgemäßen ternären Mischung auch einen oder mehrere antimikrobielle Wirkstoffe zur Verbesserung der Mundhygiene enthalten. Diese Wirkstoffe können hydrophiler, amphoterer oder hydrophober Natur sein. Beispiele solcher Wirkstoffe sind ebenfalls in WO2005/123101 genannt.

Ein erfindungsgemäßes kosmetisches, dermatologisches bzw. pharmazeutisches Produkt und/oder Mundhygieneprodukt kann neben einer erfindungsgemäßen ternären Mischung auch Farbstoffe, Färbemittel oder Pigmente enthalten, die ebenfalls in W02005/123101 genannt sind.

Kosmetische, der Mundhygiene dienende und/oder pharmazeutische Zubereitungen, die eine erfindungsgemäße ternäre Mischung enthalten, können besonders vorteilhaft Antioxidantien enthalten, wobei alle für die Mundhygiene, kosmetische und/oder dermatologische Anwendungen geeigneten oder gebräuchlichen Antioxidantien verwendet werden können. Vorteilhaft werden die Antioxidantien gewählt, die in W02005/123101 genannt sind.

Bevorzugte der Mundhygiene dienende, kosmetische und/oder pharmazeutische Zubereitungen, die eine erfindungsgemäße ternäre Mischung enthalten, enthalten auch ein oder mehrere Vitamine und/oder Vitaminvorstufen, wobei alle für kosmetische und/oder dermatologische Anwendungen geeigneten oder gebräuchlichen Vitamine und Vitaminvorstufen verwendet werden können. Hierzu zählen insbesondere die bereits in WO2005/123101 genannten Vitamine und Vitaminvorstufen.

Zur Anwendung in der für Kosmetika und Dermatika üblichen Weise werden die erfindungsgemäßen ternären Mischungen auf die Haut und/oder die Haare in ausreichender Menge aufgebracht. Besondere Vorteile bieten dabei kosmetische und dermatologische Zubereitungen, die eine erfindungsgemäße ternäre Mischung enthalten und zusätzlich als Sonnenschutzmittel wirken. Vorteilhaft enthalten diese Zubereitungen mindestens einen UVA-Filter und/oder mindestens einen UVB-Filter und/oder mindestens ein anorganisches Pigment. Die Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol bilden.

Erfindungsgemäße Zubereitungen im Bereich Kosmetika und Dermatika, die eine erfindungsgemäße ternäre Mischung enthalten, werden besonders vorteilhaft mit Substanzen kombiniert, die UV-Strahlung absorbieren oder reflektieren, namentlich für kosmetische oder hautschützende Zwecke (also nicht für mundhygienische Zwecke), wobei die Gesamtmenge der Filtersubstanzen von 0,01 Gew.-% bis 40 Gew.-%, vorzugsweise 0,1% bis 10 Gew.-%, insbesondere 1,0 bis 5,0 Gew.-% beträgt, bezogen auf das Gesamtgewicht der Zubereitungen, um kosmetische Zubereitungen zur Verfügung zu stellen, die das Haar bzw. die Haut vor ultravioletter Strahlung schützen. Vorteilhaft enthalten diese Zubereitungen mindestens einen UVA-Filter und/oder mindestens einen UVB-Filter und/oder mindestens ein anorganisches Pigment, so dass ein Lichtschutzfaktor von zumindest > 2 (bevorzugt > 5) erreicht wird. Diese erfindungsgemäßen Zubereitungen können dabei in verschiedenen Formen vorliegen, wie sie z.B. üblicherweise für Sonnenschutzzubereitungen eingesetzt werden. So können sie z.B. eine Lösung, eine Emulsion vom Typ Wasser-in-Öl (W/O) oder vom Typ Öl-in-Wasser (O/W), oder eine multiple Emulsion, beispielsweise vom Typ Wasser-in-Öl-in-Wasser (W/O/W), ein Gel, eine Hydrodispersion, einen festen Stift oder auch ein Aerosol darstellen.

Vorteilhafte UV-Filter und anorganische Lichtschutzpigmente sind genannt in WO2005/123101. Besonders zur Kombination geeignete UV-Absorber sind ebenfalls genannt in W02005/123101.

Vorteilhafte anorganische Lichtschutzpigmente sind feindisperse Metalloxide und Metallsalze die ebenfalls in WO2005/123101 genannt sind. Die Gesamtmenge an anorganischen Pigmenten, insbesondere hydrophoben anorganischen Mikropigmenten in den fertigen kosmetischen oder dermatologischen Formulierungen liegt vorteilhaft im Bereich von 0,1 bis 30 Gew.-%, bevorzugt 0,1 bis 10,0, insbesondere 0.5 bis 6.0 Gew.-%, bezogen auf das Gesamtgewicht der Formulierungen.

In manchen Zubereitungen kann auch eine Kombination mit (Metall)-Chelatoren vorteilhaft sein. Bevorzugt einzusetzende (Metall)-Chelatoren sind die in WO2005/123101 genannten Verbindungen.

Erfindungsgemäß bevorzugte kosmetische, pharmazeutische und/oder mundhygienische Zubereitungen können auch entzündungshemmende und/oder rötungs- und/oder juckreizlindernde Wirkstoffe enthalten. Es können hierbei

alle für kosmetische und/oder dermatologische und/oder der Mundhygiene dienenden Anwendungen geeigneten entzündungshemmenden bzw. rötungs- und/oder juckreizlindernden Wirkstoffe verwendet werden. Vorteilhaft werden die in WO2005/123101 genannten Verbindungen als entzündungshemmende bzw. rötungs- und/oderjuckreizlindernde Wirkstoffe verwendet.

Die Menge der Antiirritantien (eine oder mehrere Verbindungen) in den erfindungsgemäßen Zubereitungen beträgt vorzugsweise 0,0001 bis 20 Gew.-%, besonders bevorzugt 0,0001 - 10 Gew.-%, insbesondere 0,001 - 5 Gew.-%, bezogen auf das Gesamtgewicht der Zubereitung.

Erfindungsgemäße ternäre Mischungen können zu kosmetischen und/oder dermatologischen Zwecken ebenfalls vorteilhaft in Kombination mit hautaufhellenden Wirkstoffen eingesetzt werden. Erfindungsgemäß können hierbei alle für kosmetische und/oder dermatologische Anwendungen geeigneten oder gebräuchlichen hautaufhellenden Wirkstoffe verwendet werden, die in WO2005/123101 genannt sind.

Erfindungsgemäße ternäre Mischungen können insbesondere in kosmetischen und dermatologischen Zubereitungen vorteilhaft in Kombination mit Insektrepellents wie z.B. DEET, IR 3225, Dragorepel (Symrise GmbH & Co. KG) eingesetzt werden.

Weiterhin können erfindungsgemäße ternäre Mischungen insbesondere in kosmetischen und dermatologischen Zubereitungen vorteilhaft in Kombination mit Haarwachstumsinhibitoren wie in WO2005/123101 genannt eingesetzt werden.

In zahlreichen Fällen können erfindungsgemäße ternäre Mischungen auch vorteilhaft in Kombination mit den in WO2005/123101 genannten Osmolyten eingesetzt werden.

Erfindungsgemäße ternäre Mischungen können insbesondere in kosmetischen und dermatologischen Zubereitungen vorteilhaft in Kombination mit Haarpflegemitteln und Antischuppenwirkstoffen (z.B. Climbazol, Ketoconazol, Piroctonoleamin, Zink-Pyrithion) eingesetzt werden.

Auch können erfindungsgemäße ternäre Mischungen in zahlreichen Fällen vorteilhaft in Kombination mit einem oder mehreren Konservierungsmitteln in erfindungsgemäßen Zubereitungen eingesetzt werden. Vorzugsweise gewählt werden hierbei die in WO2005/123101 genannten Konservierungsmittel.

Zudem ist es erfindungsgemäß bevorzugt, erfindungsgemäße ternäre Mischungen in Kombination mit Substanzen einzusetzen, die vornehmlich zur Inhibition des Wachstums unerwünschter Mikroorganismen auf oder in tierischen Organismen eingesetzt werden. Erwähnenswert sind insoweit neben klassischen Konservierungsmitteln als weitere Wirkstoffe neben der großen Gruppe der klassischen Antibiotika insbesondere die in W02005/123101 genannten Verbindungen.

Darüber hinaus können erfindungsgemäße ternäre Mischungen erfindungsgemäß auch in Kombination mit schweißhemmenden Wirkstoffen (Antitranspirantien) besonders vorteilhaft zur Bekämpfung von Körpergeruch in kosmetischen, dermatologischen und/oder pharmazeutischen Zubereitungen eingesetzt werden. Als schweißhemmende Wirkstoffe kommen vor allem die in WO2005/123101 genannten Verbindungen in Betracht.

Erfindungsgemäße ternäre Mischungen können in kosmetischen und dermatologischen Zubereitungen vorteilhaft mit kosmetischen Hilfsstoffen kombiniert werden, wie sie üblicherweise in solchen Zubereitungen verwendet werden, also z.B. mit: Parfümölen; Mitteln zum Verhindern des Schäumens; Farbstoffen; Pigmenten, die eine färbende Wirkung haben; Verdickungsmittel; oberflächenaktiven Substanzen; Emulgatoren; weichmachenden Substanzen; anfeuchtenden und/oder feuchthaltenden Substanzen; Fetten; Ölen; Wachsen; anderen üblichen Bestandteilen einer kosmetischen Formulierung wie Alkoholen, Polyolen, Polymeren, Schaumstabilisatoren, Elektrolyten, organischen Lösungsmitteln oder Silikonderivaten.

In erfindungsgemäße ternäre Mischungen enthaltende Formulierungen zur topischen prophylaktischen oder kosmetischen Behandlung der Haut ist regelmäßig ein hoher Anteil an pflegenden Substanzen vorteilhaft. Gemäß einer bevorzugten Ausführungsform enthalten die Zusammensetzungen ein oder mehrere der in WO2005/123101 genannten pflegenden tierischen und/oder pflanzlichen Produkte.

Der erfindungsgemäßen ternären Mischung können vorteilhaft auch die in WO2005/123101 genannten tierischen und/oder pflanzliche Proteinhydrolysate zugesetzt werden.

Sofern eine kosmetische oder dermatologische, erfindungsgemäße ternäre Mischung enthaltende Zubereitung eine Lösung oder Lotion ist, können vorteilhafterweise die in WO2005/123101 genannten Lösungsmittel verwendet werden.

Erfindungsgemäße kosmetische Zubereitungen, die eine erfindungsgemäße ternäre Mischung enthalten, können vorteilhafterweise auch die in WO2005/123101 genannten genannten Feuchthalteregulatoren enthalten.

Kosmetische Zubereitungen, die eine erfindungsgemäße ternäre Mischung enthalten, können in bevorzugten Ausführungsformen der Erfindung auch Mono-, Di- und Oligosaccharide enthalten.

Darüber hinaus ist es bevorzugt, wenn kosmetische Zubereitungen, die eine erfindungsgemäße ternäre Mischung enthalten, einen oder mehrere weitere Pflanzenextrakte enthalten, die üblicherweise durch Extraktion der gesamten Pflanze, in einzelnen Fällen aber auch ausschließlich aus Blüten und/oder Blättern, Holz, Rinde oder Wurzeln der Pflanze hergestellt werden. Hinsichtlich der für kosmetische, pharmazeutische und/oder dermatologisch Zwecke

verwendbaren Pflanzenextrakte wird der Fachmann die in der auf Seite 44 der 3. Auflage des Leitfadens zur Inhaltsstoffdeklaration kosmetischer Mittel, herausgegeben vom Industrieverband Körperpflegemittel und Waschmittel e.V. (IKW), Frankfurt beginnenden Tabelle aufgeführten Inhaltsstoffe in Betracht ziehen. Vorteilhaft sind für kosmetische, dermatologische, pharmazeutische und/oder der Mundhygiene dienende Zwecke insbesondere die in WO2005/123101 genannten Extrakte geeignet.

Kosmetische Zubereitungen, die eine erfindungsgemäße ternäre Mischung enthalten, können, insbesondere wenn kristalline oder mikrokristalline Festkörper, beispielsweise anorganische Mikropigmente in die Zubereitungen eingearbeitet werden sollen, erfindungsgemäß auch in WO2005/123101 genannte anionische, kationische, nichtionische und/oder amphotere Tenside enthalten.

Die oberflächenaktive Substanz kann in einer Konzentration zwischen 1 und 98 Gew.-% in den erfindungsgemäßen Zubereitungen vorliegen, bezogen auf das Gesamtgewicht der Zubereitungen.

Kosmetische oder dermatologische Zubereitungen, die eine erfindungsgemäße ternäre Mischung enthalten, können auch als Emulsionen vorliegen.

Die Ölphase erfindungsgemäßer kosmetischer, dermatologischer und/oder pharmazeutischer Zubereitungen kann vorteilhaft gewählt werden aus den in WO2005/123101 genannten Substanzgruppen.

Als Emulsion vorliegende erfindungsgemäße Zubereitungen umfassen vorteilhaft einen oder mehrere Emulgatoren. O/W-Emulgatoren können beispielsweise vorteilhaft gewählt werden aus der Gruppe der in WO2005/123101 genannten Produkte.

Bevorzugte Ausgestaltungen und weitere Aspekte der vorliegenden Erfindung ergeben sich aus den beigefügten Patentansprüchen und den nachfolgenden Beispielen zusammen mit den Tabellen, wobei die Beispiele die Erfindung nicht beschränken sollen:

Beispiel 1: Verfahren zur Bestimmung der Collagensynthese steigernden Wirksamkeit ausgewählter Substanzen natürlichen oder synthetischen Ursprungs.

Verantwortlich für die Collagensynthese in der Dermis-Schicht der Haut sind Fibroblasten. Als Testsystem werden Normal Humane Dermalen Fibroblasten (NHDF) in einer so genannten Monolayer-Kultur in 96-Well-MTPs kultiviert, über einen definierten Zeitraum mit Testsubstanzen inkubiert und anschließend die Collagenzunahme qualitativ über eine Färbemethode bestimmt.

Das Assay-Prinzip basiert auf der histologischen, spezifischen Anfärbung von löslichem Collagen (vorwiegend Collagen Typ I und III) mit Hilfe des Farbstoffes Sirius Red (Direct Red 80). Sirius Red ist ein anionischer Farbstoff an dessen Seitenketten Sulphonsäuregruppen angelagert sind. Diese Sulphonsäuregruppen reagieren mit den Seitenkettengruppen der im Collagen vorwiegend vorkommenden Aminosäuren (Prolin, Hydroxyprolin, Lysin und Hydroxylysin), wobei eine intakte Collagenstruktur gegeben sein muss.

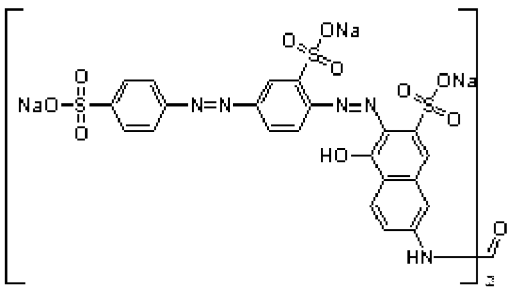

Sirius Red

$C_{45}H_{26}N_{10}Na_6O_{21}S_6$

Als Vorlage für das Assay-Design dient die bei B.J.Walsh et al., Analytical Biochemistry 203, 187-190 (1992) beschriebene Vorgehensweise zur quantitativen Bestimmung von Collagen im 96-Well Maßstab.

Die Collagensynthese steigernde Wirkung der Testsubstanzen wird an in vitro gealterten (>8. Passage) NHDF in 96-Well Mikrotiterplatten ($2*10^4$ Zellen pro Well) getestet. Für jedes Prüfmuster muss zuvor ein geeignetes Lösungsmittel ermittelt werden, in dem eine Stocklösung des Prüfmusters angesetzt wird. Die Inkubationslösungen der Testsubstanzen werden anschließend in mit 0,2% BSA supplementiertem DMEM hergestellt. Mögliche Lösungsmittel sollten in allen Verdünnung der Inkubationslösung max. 0,2 %ig vorliegen. Die maximale Einsatzkonzentration der Testsubstanzen resultiert aus den in der Zytotoxizitäts-Untersuchung (Resazurin-Assay an 3T3 Maus Fibroblasten) gewonnen $IC_{20}$-Werten und ist auf $0,1*IC_{20}$ festgelegt.

Nach einer 24h-igen Adaptionsphase der NHDF werden die Zellen für 48 h mit den Inkubationslösungen und

entsprechenden Kontroll-Lösungen (Lösungsmittel und Blank) inkubiert (37°C, 5% $CO_2$). Um mögliche Einflüsse der Testsubstanzen auf den später folgenden Färbevorgang erkennen zu können, wird jeweils die höchste Einsatzkonzentration der Testsubstanzen auch ohne Zellen als Background-Kontrolle mitgeführt. Anschließend erfolgt ein Zellaufschluss durch einen 2-fachen Einfrier-/Auftau-Zyklus (-20°C/-80°C/37°C/-80°C/37°C; je 10 Minuten) und eine 96 stündige Eindampfphase (24h feuchte Wärme bei 37°C, 72h trockene Wärme bei 30°C). Die so erhaltenen Collagen-beschichteten Wells können mit dem Farbstoff Sirius Red (0,1 %ige Lösung) angefärbt werden. Der an das Collagen gebundene Farbstoff wird durch einen dreifachen Waschschritt mit 0,01 mM HCl-Lösung und anschließender Inkubation (min. 5 Minuten) mit 0,1 mM NaOH-Lösung wieder in Lösung gebracht. Die Absorption der entstandenen Farblösung kann photometrisch bei 540 nm ermittelt werden.

Aufgrund des linearen Zusammenhangs zwischen Collagengehalt und aus der Färbung resultierenden OD-Werten, wird für die qualitative Bewertung der Collagensynthese-steigernden Wirkung die OD-Erhöhung bestimmt. Hierfür werden die Mittelwerte und Standardabweichungen der Kontrollen und der Proben berechnet und anschließend eine Background bzw. Blank-Korrektur vorgenommen. Die OD-Erhöhung ergibt sich dann aus der Relation zwischen behandelten (mit Testsubstanz) und unbehandelten (nur LM oder Medium) Zellen.

Quellen:

1. Walsh B.J., Thornton S.C., Penny R. and Breit S.N.: Microplate Reader-Based Quantitation of Collagens: Analytical Biochemistry 203, 187-190 (1992)

2. Nethery A., Lyons J.G. and O'Grady R.L.: A Spectrometric Collagenase Assay: Analytical Biochemistry 159, 390-395 (1986)

Beispiel 2: Bestimmung der synergistisch verstärkten Collagensynthese steigernden Wirksamkeit ausgewählter ternärer Mischungen enthaltend mindestens einen Extrakt aus einer Aloe Art, mindestens einen Stoff aus der Gruppe Ascorbinsäure und deren Derivate und mindestens einen Rosaceen-Extrakt, vorzugsweise Himbeerblattextrakt, Erdbeerblattextrakt, Brombeerblattextrakt oder Mädesüßkrautextrakt.

Zur Bestimmung potentiell synergistisch verstärkter Wirksamkeiten ternärer Mischungen enthaltend Aloe Extrakt, mindestens einen Stoff aus der Gruppe Ascorbinsäure und deren Derivate und mindestens einen Rosaceen-Extrakt, vorzugsweise Himbeerblattextrakt, Erdbeerblattextrakt, Brombeerblattextrakt oder Mädesüßkrautextrakt wurden sowohl für die Einzelstoffe als auch für verschiedene ternäre Mischungen die Collagensynthese steigernden Eigenschaften gemäß der in Beispiel 1 beschriebenen Versuchsdurchführung ermittelt. Die Ergebnisse der Untersuchungen sind in Tabelle 1 gelistet.

Tabelle 1:

| Collagensynthese Steigerung von Magnesium Ascorbylphosphat, Aloe Extrakt, Himbeerblattextrakt, Erdbeerblattextrakt, Mädesüßkrautextrakt sowie ternäre Mischungen enthaltend (i) Magnesium Ascorbylphosphat, Aloe Extrakt und Mädesüßkrautextrakt, (ii) Magnesium Ascorbylphosphat, Aloe Extrakt und Erdbeerblattextrakt, (iii) Magnesium Ascorbylphosphat, Aloe Extrakt und Himbe erblattextrakt sowie (iiii) Magnesium Ascorbylphosphat, Aloe Extrakt und Brombeerblattextrakt ; Steigerung in % bezogen auf unbehandelte Kontrolle | | | |
|---|---|---|---|
| | Einsatzkonzentration [mg/ml] | Collagensynthese Steigerung [%], bezogen auf unbehandelte Kontrolle | Synergie-Index SI |
| Aloe Extrakt (Bio505) | 10.860 | 45.70 | ./. |
| Magnesium Ascorbylphosphat (Bio172) | 0.362 | 66.97 | ./. |
| Mädesüßkrautextrakt (Bio1544) | 0.030 | 14.22 | ./. |
| **Ternäre Mischung (Bio505 + Bio172 + Bio1544)** | **3.620 (Bio505) + 0.121 (Bio 172) + 0.010 (Bio1544)** | **112.76** | **3.99** |

(fortgesetzt)

| Collagensynthese Steigerung von Magnesium Ascorbylphosphat, Aloe Extrakt, Himbeerblattextrakt, Erdbeerblattextrakt, Mädesüßkrautextrakt sowie ternäre Mischungen enthaltend (i) Magnesium Ascorbylphosphat, Aloe Extrakt und Mädesüßkrautextrakt, (ii) Magnesium Ascorbylphosphat, Aloe Extrakt und Erdbeerblattextrakt, (iii) Magnesium Ascorbylphosphat, Aloe Extrakt und Himbe erblattextrakt sowie (iiii) Magnesium Ascorbylphosphat, Aloe Extrakt und Brombeerblattextrakt ; Steigerung in % bezogen auf unbehandelte Kontrolle | | | |
|---|---|---|---|
| | Einsatzkonzentration [mg/ml] | Collagensynthese Steigerung [%], bezogen auf unbehandelte Kontrolle | Synergie-Index SI |
| | Einsatzkonzentration [mg/ml] | Collagensynthese Steigerung [%], bezogen auf unbehandelte Kontrolle | Synergie-Index SI |
| Aloe Extrakt (Bio505) | 10.860 | 45.70 | ./. |
| Magnesium Ascorbylphosphat (Bio172) | 0.362 | 66.97 | ./. |
| Erdbeerblattextrakt (Bio1545) | 0.030 | 14.85 | ./. |
| **Ternäre Mischung (Bio505 + Bio172 + Bio1545)** | **3.620 (Bio505) + 0.121 (Bio 172) + 0.010 (Bio1545)** | **96.37** | **3.31** |
| | Einsatzkonzentration [mg/ml] | Collagensynthese Steigerung [%], bezogen auf unbehandelte Kontrolle | Synergie-Index SI |
| Aloe Extrakt (Bio505) | 10.860 | 47.59 | ./. |
| Magnesium Ascorbylphosphat (Bio172) | 0.362 | 67.44 | ./. |
| Himbeerblattextrakt (Bio 615) | 0.030 | 16.74 | ./. |
| **Ternäre Mischung (Bio505 + Bio172 + Bio615)** | **3.620 (Bio505) + 0.121 (Bio 172) + 0.010 (Bio615)** | **142.15** | **4.48** |
| | Einsatzkonzentration [mg/ml] | Collagensynthese Steigerung [%], bezogen auf unbehandelte Kontrolle | Synergie-Index SI |
| Magnesium Ascorbylphosphat (Bio172) | 0.362 | 66.97 | ./. |
| Aloe Extrakt (Bio505) | 10.860 | 45.70 | ./. |
| Brombeerblattextrakt (Bio684/4) | 0.003 | 14.15 | ./. |

(fortgesetzt)

| | Einsatzkonzentration [mg/ml] | Collagensynthese Steigerung [%], bezogen auf unbehandelte Kontrolle | Synergie-Index SI |
|---|---|---|---|
| **Ternäre Mischung (Bio505 + Bio172 + Bio684/4)** | **3.620 (Bio505) + 0.121 (Bio 172) + 0.010 (Bio684/4)** | **153.93** | **5.46** |

Ergebnis:

Die Berechung der Synergieindices (SI-Werte) der erfindungsgemäßen ternären Mischungen nach Kull (Literatur: F.C.Kull et al, Applied Microbiology Vol.9, p. 538-541 (1961); D.C.Steinberg, Cosmetics & Toiletries Vol.115(11), 59-62 (2000) erfolgte mittels folgender Gleichung.

$$SI = Z * D/A + Z * E/B + Z * F/C$$

wobei:

D,E,F : Faktor Mengenanteil der Einzelbestandteile (Bsp: 33 Gewichtsprozent = Faktor 0.33)

A, B, C: Collagenstimulation der Einzelstoffe (Zunahme in %, bezogen auf wirkstofffreie Kontrolle)

Z: Collagenstimulation der ternären Mischung (Zunahme in %, bezogen auf wirkstofffreie Kontrolle)

Ein synergistischer Effekt liegt vor, wenn Werte größer 1.00 erhalten werden. Werte kleiner 1.00 zeigen einen antagonistischen Effekt. Bei einem Wert von exakt 1.00 liegt weder ein synergistischer noch ein antagonistischer Effekt vor.

Nachfolgend ist ein Rechenbeispiel für die ternäre Mischung enthaltend jeweils ein Drittel (Faktor D, E und F: jeweils 0.33) der im Einzelexperiment verwendeten Mengen an Magnesium Ascorbylphosphat, Aloe Extrakt und Himbeerblattextrakt aufgeführt.

$$SI = 142.15 * 0.33/67.44 + 142.15 * 0.33/47.59 + 142.15 * 0.33/16.74 = 4.48$$

Der SI-Wert von 4.48 zeigt eine signifikante, synergistisch verstärkte Wirkung der Mischung enthaltend Magnesium Ascorbylphosphat, Aloe Extrakt und Himbeerblattextrakt an. Die SI-Werte der im weiteren untersuchten ternären Mischung sind in Tabelle 1 zusammengefasst.

Die in Tabelle 1 gelisteten Ergebnisse und hier insbesondere die errechneten Synergie Indices zeigen eindeutig, dass die erfindungsgemäßen ternären Mischungen enthaltend: Aloe Extrakt, Magnesium Ascorbylphosphat und einen Rosaceen-Extrakt (gemäß Tabelle 1 z.B. Himbeerblattextrakt, Erdbeerblattextrakt, Brombeerblattextrakt oder Mädesüßkrautextrakt) bei einem Mengenverhältnis von 96.51 Gewichtsprozent Aloe zu 3.22 Gewichtsprozent Magnesium Ascorbylphosphat zu 0.27% Gewichtsprozent Rosaceen-Extrakt eine synergistisch verstärkte Collagensynthese steigernde Wirksamkeit aufweisen. Im Fall der ternären Mischung enthaltend Aloe Extrakt, Magnesium Ascorbylphosphat und Erdbeerblattextrakt konnte die Collagensynthese bei Einsatz von jeweils einem Drittel der Gesamtmenge um 96.37% gesteigert werden (Synergie Index: 3.31). Im Fall der ternären Mischung enthaltend Aloe Extrakt, Magnesium Ascorbylphosphat und Mädesüßkrautextrakt konnte die Collagensynthese bei Einsatz von jeweils einem Drittel der Gesamtmenge um 112.76% gesteigert werden (Synergie Index: 3.99). Die höchste Steigerung der Collagensynthese um 142.15% bzw. 153.93% konnte bei Einsatz von jeweils einem Drittel der Gesamtmenge für die ternäre Mischung enthaltend Aloe Extrakt, Magnesium Ascorbylphosphat und Himbeerblattextrakt bzw. Brombeerblattextrakt erzielt werden. (Synergie Indices: 4.48 und 5.46). Da in allen Fällen Synergie Indices größer 1 ermittelt wurden, ist der synergistische Effekt der erfindungsgemäßen ternären Mischungen eindeutig bewiesen.

Beispiel 3: Bestimmung der synergistisch verstärkten Collagensynthese steigernden Wirksamkeit weiterer ternärer Mischungen enthaltend (i) Aloe Extrakt, (ii) Magnesium Ascorbylphospat oder Natrium Ascorbylphosphat und (iii) Himbeerblattextrakt:

Beispiel 3 zeigt weitere ternäre Mischungen mit gegenüber Beispiel 2 geänderten Mischungsverhältnissen, bei denen ebenfalls eine synergistisch verstärkte Wirksamkeiten der ternären Mischungen nachgewiesen werden konnte. Die Ergebnisse der Untersuchungen sind in Tabelle 2 gelistet.

Tabelle 2: Collagensynthese Steigerung von Aloe Extrakt, Magnesium Ascorbylphosphat, Natrium Ascorbylphosphat, Himbeerblattextrakt sowie ternäre Mischung enthaltend (i) Aloe Extrakt, (ii) Magnesium Ascorbylphosphat oder Natrium Ascorbylphosphat und (iii) Himbeerblattextrakt; Steigerung in % bezogen auf unbehandelte Kontrolle.

| | Einsatzkonzentration [mg/ml] | Collagensynthese Steigerung [%], bezogen auf unbehandelte Kontrolle | Synergie-Index SI |
|---|---|---|---|
| Aloe Extrakt (Bio505) | 10.110 | 39.87 | ./. |
| Magnesium Ascorbylphosphat (Bio1300/1) | 1.020 | 141.13 | ./. |
| Natrium Ascorbylphosphat (Bio1586) | 1.020 | 144.57 | ./. |
| Himbeerblattextrakt (Bio 615) | 0.12 | 9.93 | ./. |
| Ternäre Mischung A (Bio505 + Bio1300/1+Bio615) | 3.370 (Bio505) + 0.340 (Bio ...) + 0.040 (Bio615) | 160.52 | 7.04 |
| Ternäre Mischung B (Bio505 + Bio1586 + Bio615) | 3.370 (Bio505) + 0.340 (Bio ...) + 0.040(Bio615) | 94.95 | 4.16 |

Ergebnis:

Die in Tabelle 2 gelisteten Ergebnisse zeigen, dass weitere erfindungsgemäße ternäre Mischungen enthaltend: Aloe Extrakt, Magnesium Ascorbylphosphat, und Himbeerblattextrakt bzw. Aloe Extrakt, Natrium Ascorbylphosphat und Himbeerblattextrakt in einem Mengenverhältnis von 90 Gewichtsprozent Aloe Extrakt zu 9 Gewichtsprozent Magnesium Ascorbylphosphat (bzw. 9 Gewichtsprozent Natrium Ascorbylphosphat) zu 1 Gewichtsprozent Himbeerblattextrakt ebenfalls eine synergistisch verstärkte, die Collagensynthese steigernde Wirksamkeit aufweisen. Im Fall der ternären Mischung enthaltend Aloe Extrakt, Magnesium Ascorbylphosphat und Himbeerblattextrakt konnte die Collagensynthese bei Einsatz von jeweils einem Drittel der getesteten Menge an Einzelstoffen um 160.52% (Synergie Index: 7.04) gesteigert werden. Im Fall der ternären Mischung enthaltend (ii) Aloe Extrakt, Natrium Ascorbylphosphat und Himbeerblattextrakt konnte bei Einsatz von jeweils einem Drittel der getesteten Menge an Einzelstoffen die Collagensynthese um 94.95% (Synergie Index 4.16) gesteigert werden. Da in beiden Fällen Synergie Indices größer 1 ermittelt wurden, ist der synergistische Effekt der erfindungsgemäßen ternären Mischungen eindeutig bewiesen.

Beispiel 4: weitere kosmetische/dermatologische Anwendungsbeispiele

Zur Anwendung werden die synergistisch wirksame ternäre Mischungen enthaltenden dermatologischen und kosmetischen Zubereitungen in Kombination mit anderen kosmetischen Wirk- und Zusatzstoffen in der für Kosmetika üblichen Weise auf die Haut und/oder die Haare in ausreichender Menge aufgebracht. Nachfolgend sind beispielhaft vorteilhafte Zubereitungen für einige Anwendungen angegeben:

4.1 Antifalten W/O Creme

| Teil | Rohstoffname (Hersteller) | INCI-Bezeichnung | Gehalt in Gew.-% |
|---|---|---|---|
| A | Dragosan W/O P (Symrise) | Sorbitan Isostearate, Hydrogenated Castor Oil, Ceresin, Beeswax (Cera Alba) | 8.00 |
| | Dragoxat EH (Symrise) | Ethylhexyl Ethylhexanoate | 8.00 |
| | Isodragol (Symrise) | Triisononanoin | 8.00 |
| | Dow Corning 200 Fluid (300 cs) (Dow corning) | Dimethicone | 2.00 |
| | Betone Gel Mio (Elementis) | Mineral Oil, Quaternium-18 Hectorite, Propylene Carbonate | 3.00 |
| B | Demineralisiertes Wasser | Wasser (Aqua) | add 100 |
| | Magnesiumsufat Hepta Hydrat (Merck) | Magnesium Sulfate | 1.00 |
| | Glycerin, 99.5% | Glycerin | 3.00 |
| | Drago-Beta-Glucan (Symrise) | Water (Aqua), Butylene Gylcol, Glycerin, Avena Sativa (Oat) Kernel Extract | 5.00 |
| | **Himbeerblatt-Extrakt** | | **0.005** |
| | **Aloe Extrakt** | | **0.450** |
| | **Magnesium Ascorbylphosphat** | | **0.045** |
| | Dragocid Liquid (Symrise) | Phenoxyethanol, Methylparaben, Ethylparaben, Butylparaben, Propylparaben, Isobutylparaben | 0.80 |
| C | Symrise Parfümöl | Fragrance | 0.30 |

Teil A und B ohne Drago-Beta-Glucan auf 75°C erhitzen. Drago-Beta-Glucan zu Teil B geben. Teil B zu Teil A geben und emulgieren. Emulsion kaltrühren, bei ca. 50°C nochmals homogenisieren und bei ca. 35°C Parfümöl zugeben.

4.2 Nachtcreme gegen Hautalterung

| Teil | Rohstoffname (Hersteller) | INCI-Bezeichnung | Gehalt in Gew.-% |
|---|---|---|---|
| A | Dragosan W/O P (Symrise) | Sorbitan Isostearate, Hydrogenated Castor Oil, Ceresin, Beeswax (Cera Alba) | 6.00 |
| | PCL-Liquid (Symrise) | Cetearyl Ethylhexanoate, Isopropyl Myristate | 12.00 |
| | Sonnenblumenöl (H. Erhard Wagner) | Helianthus Annuus (Sunflower) Seed Oil | 5.00 |
| | Süßmandelöl (H. Erhard Wagner) | Prunus dulcis | 5.00 |
| | Dragosan W/O Liquid (Symrise) | Polyglyceryl-3-Polyricinoleate, Sorbitan Isostearate | 1.00 |
| | Alugel 34 TH (Baerlocher) | Aluminium Stearate | 1.00 |

(fortgesetzt)

| Teil | Rohstoffname (Hersteller) | INCI-Bezeichnung | Gehalt in Gew.-% |
|---|---|---|---|
| | Oxynex 2004 (Merck) | Bht | 0.10 |
| B | Demineralisiertes Wasser | Wasser (Aqua) | add 100 |
| | Glycerin, 99.5% | Glycerin | 2.00 |
| | Karion F (Merck) | Sorbitol | 2.00 |
| | Aloe Vera Gel Konzentrat 10/1 (Symrise) | Water (Aqua), Aloe Barbadensis Leaf Juice | 3.00 |
| | Extrapon Hamamelis Destillat Farblos (Symrise) | Propylene Glycol, Hamamelis Virginiana (Witch Hazel) Water, Water (Aqua), Hamamelis Virginiana (Witch Hazel) Extract | 1.00 |
| | **Erdbeerblatt-Extrakt** | | **0.040** |
| | **Aloe Extrakt** | | **2.000** |
| | **Natrium Ascorbylphosphat** | | **0.400** |
| | Magnesiumsufat Hepta Hydrat (Merck) | Magnesium Sulfate | 0.70 |
| | Dragocid Liquid (Symrise) | Phenoxyethanol, Methylparaben, Ethylparaben, Butylparaben, Propylparaben, Isobutylparaben | 0.80 |
| C | Vitamin E acetat (DSM Nutritional Products) | Tocopheryl Acetate | 3.00 |
| | Vitamin A-Palmitat in Öl ( 1 Mio. le/G) (DSM Nutrional Products) | Retinyl Palmitate | 0.20 |
| | -(-Alpha-)-Bisabolol, natürlich (Symrise) | Bisabolol | 0.10 |
| | Symrise Parfümöl | Fragrance | 0.40 |

Teil A und B getrennt auf ca. 80°C erhitzen. Tei B zu Teil A geben, emulgieren und kaltrühren. Bei ca. 60°C nochmals homogenisieren und bei ca. 35°C Teil C zugeben.

4.3 O/W Lotion gegen Hautalterung mit UVA/B-Breitbandschutz

| Teil | Rohstoffname (Hersteller) | INCI-Bezeichnung | Gehalt in Gew.-% |
|---|---|---|---|
| A | Emulsiphos (Symrise) | Potassium Cetyl Phospahte, Hydrogenated Palm Glycerides | 1.50 |
| | Tegosoft TN (Degussa) | C12-15 Alkyl Benzoate | 5.00 |
| | Copherol 1250 (Cognis) | Tocopheryl Acetate | 0.50 |
| | Lanette O (Cognis) | Cetearyl Alcohol | 1.00 |
| | Neutralöl (Symrise) | Caprylic/Capric Triglyceride | 2.00 |

(fortgesetzt)

| Teil | Rohstoffname (Hersteller) | INCI-Bezeichnung | Gehalt in Gew.-% |
|---|---|---|---|
| | Dow Corning 246 Fluid (Dow Corning) | Cyclohexasiloxane (and) Cyclopentasiloxane | 2.00 |
| | Neo Heliopan AV (Symrise) | Ethylhexyl Methoxycinnamate | 3.00 |
| | Neo Heliopan OS (Symrise) | Ethylhexyl Salicylate | 5.00 |
| | Neo Heliopan MBC (Symrise) | 4-Methylbenzylidene Camphor | 1.50 |
| | Neo Heliopan 357 (Symrise) | Butyl Methoxydibenzoylmethane | 1.00 |
| | EDETA DB (BASF) | Disodium EDTA | 0.10 |
| | Keltrol T (Danby-Chemie) | Xanthan Gum | 0.20 |
| | Carbopol ETD 2050 (Noveon) | Carbomer | 0.20 |
| B | Demineralisiertes Wasser | Water (Aqua) | add 100 |
| | Glycerin, 99.5% | Glycerin | 4.70 |
| | Dragocid Liquid (Symrise) | Phenoxyethanol, Methylparaben, Ethylparaben, Butylparaben, Propylparaben, Isobutylparaben | 0.70 |
| | Neo Heliopan AP (22% wässr. Lösung neutralisiert mit Triethanolamin) (Symrise) | Disodium Phenyl Dibenzimidazole Tetrasulfonate | 4.55 |
| | Neo Heliopan Hydro (30% wässr. Lösung neutralisiert mit Triethanolamin) (Symrise) | Phenylbenzimidazole Sulfonic Acid | 6.67 |
| | **Erdbeerblatt-Extrakt** | | **0.10** |
| | **Aloe Extrakt** | | **1.80** |
| | **Magnesium Ascorbylphosphat** | | **0.60** |
| C | Triethanolamin, 99% | Triethanolamine | 0.50 |
| D | Symrise Parfümöl | Fragrance | 0.40 |
| | Dragosantol (Symrise) | Bisabolol | 0.10 |

Teil A (bis auf Keltrol und Carbopol) auf 85°C erhitzen. Keltrol und Carbopol zugeben und homogenisieren. Teil B auf 85 °C erhitzen und Teil B zu Teil A geben. Teil C direkt zu Teil A/B geben, homogenisieren und abkühlen lassen. Teil D zu Teil A/B/C geben und homogenisieren. Der pH-Wert des Endprodukts sollte bei ca. 7,2 bis 7,5 liegen.

4.4 After Sun Lotion (O/W) gegen Hautalterung

| Teil | Rohstoffname (Hersteller) | INCI-Bezeichnung | Gehalt in Gew.-% |
|---|---|---|---|
| A | Demineralisiertes Wasser | Water (Aqua) | add 100 |

(fortgesetzt)

| Teil | Rohstoffname (Hersteller) | INCI-Bezeichnung | Gehalt in Gew.-% |
|---|---|---|---|
| | Glycerin, 99.5% | Glycerin | 4.00 |
| | D-Panthenol (BASF) | Panthenol | 1.00 |
| | Butylenglycol | Butylene Glycol | 5.00 |
| | Allantoin (Merck) | Allantoin | 0.10 |
| | Aloe Vera Gel Konzentrat 10/1 (Symrise) | Water (Aqua), Aloe Barbadensis Leaf Juice | 3.0 |
| | **Himbeerblatt-Extrakt** | | **0.10** |
| | **Aloe Extrakt** | | **1.50** |
| | **Natrium Ascorbylphosphat** | | **0.50** |
| | Lara Care A-200 (Rahn) | Galactoarabinan | 0.25 |
| B | Baysilone Oil M 350 (GE Bayer Silicones) | Dimethicone | 1.00 |
| | Copherol 1250 (Cognis) | Tocopheryl Acetate | 0.50 |
| | Tegosoft TN (Degussa) | C12-15 Alkyl Benzoate | 5.00 |
| | Cetiol SB 45 (Cognis) | Butyrospermum Parkii (Shea Butter) | 1.00 |
| | Cetiol OE (Cognis) | Dicaprylyl Ether | 4.00 |
| | -(-Alpha-)-Bisabolol, natürlich (Symrise) | Bisabolol | 0.10 |
| | Dragocid Liquid (Symrise) | Phenoxyethanol, Methylparaben, Ethylparaben, Butylparaben, Propylparaben, Isobutylparaben | 0.70 |
| | Pemulen TR-2 (Noveon) | Acrylates/C10-30 Alkyl Acrylate Crosspolymer | 0.25 |
| | Frescolat ML crist. (Symrise) | Menthyl Lactate | 0.80 |
| | Symrise Parfümöl | Fragrance | 0.30 |
| C | Natriumhydroxid (10% wässr. Lösung) | Sodium Hydroxide | 0.60 |

Teil A in Wasser lösen. Frescolat ML crist. und Cetiol SB 45 von Teil B unter Erhitzen auf maximal 35°C in Tegosoft TN lösen, abkühlen lassen und die restlichen Bestandteile von Teil B zugeben. Teil B unter Rühren zu Teil A geben und homogenisieren. Der pH-Wert des Endprodukts sollte bei ca. 6,0 liegen.

4.5 Bodyspray O/W gegen Hautalterung

| Teil | Rohstoffname (Hersteller) | INCI-Bezeichnung | Gehalt in Gew.-% |
|---|---|---|---|
| A | Dracorin GOC (Symrise) | Glyceryl Oleate Citrate, Caprylic/Capric Triglyceride | 2.00 |
| | Neutralöl | Caprylic/Capric Triglyceride | 4.00 |
| | Paraffinöl 5 Grad E (Parafluid) | Paraffinum Liquidum | 4.00 |

(fortgesetzt)

| Teil | Rohstoffname (Hersteller) | INCI-Bezeichnung | Gehalt in Gew.-% |
|---|---|---|---|
| | PCL Liquid 100 (Symrise) | Cetearyl Ethylhexanoate | 7.00 |
| | Dragoxat EH (Symrise) | Ethylhexyl Ethylhexanoate | 4.00 |
| | Dow Corning 345 Fluid (Dow Corning) | Cyclomethicone | 0.50 |
| | Dragosantol (Symrise) | Bisabolol | 0.10 |
| | Symrise Parfümöl | Fragrance | 0.20 |
| **B** | Demineralisiertes Wasser | Wasser (Aqua) | add 100 |
| | Pemulen TR-2 (Noveon) | Acrylates/C10-30 Alkyl Acrylate Crosspolymer | 0.20 |
| | Hydrolite-5 (Symrise) | Pentylene Glycol | 5.00 |
| | Drago-Oat-Active (Symrise) | Water (Aqua), Butylene Gylcol, Avena Sativa (Oat) Kernel Extract | 1.00 |
| | **Himbeerblatt-Extrakt** | | **0.001** |
| | **Aloe Extrakt** | | **0.990** |
| | **Nastrium Ascor-bylphosphat** | | **0.020** |
| **C** | Natriumhydroxid (10% wässr. Lösung) | Sodium Hydroxide | 0.40 |

Pemulen in Wasser quellen, dann die restlichen Rohstoffe von Teil B zugeben. Teil A mischen. Teil B ohne Rühren zu Teil A geben, erst dann emulgieren. Teil C während des Emulgierens zugeben. Der pH-Wert des Endprodukts sollte bei ca. 6,3 liegen.

4.6 O/W Creme gegen Hautalterung

| Teil | Rohstoffname (Hersteller) | INCI-Bezeichnung | Gehalt in Gew.-% |
|---|---|---|---|
| **A** | Dracorin GMS (Symrise) | Glyceryl Stearate | 2.00 |
| | PCL-Solid (Symrise) | Stearyl Heptanoate, Stearyl Caprylate | 2.00 |
| | Lanette O (Cognis) | Cetearyl Alcohol | 3.00 |
| | PCL Liquid 100 (Symrise) | Cetearyl Ethylhexanoate | 5.00 |
| | Isodragol (Symrise) | Triisononanoin | 2.00 |
| | Abil 350 (Degussa-Goldschmidt) | Dimethicone | 2.00 |
| | Dragoxat EH (Symrise) | Ethylhexyl Ethylhexanoate | 3.00 |
| **B** | Demineralisiertes Wasser | Wasser (Aqua) | add 100 |
| | Carbopol Ultrez-10 (Nove-on) | Carbomer | 0.10 |
| | Keltrol RD (CP-Kelco) | Xanthan Gum | 0.10 |

(fortgesetzt)

| Teil | Rohstoffname (Hersteller) | INCI-Bezeichnung | Gehalt in Gew.-% |
|---|---|---|---|
| | Emulsiphos (Symrise) | Potassium Cetyl Phospahte, Hydrogenated Palm Glycerides | 2.00 |
| | Dragocid Liquid (Symrise) | Phenoxyethanol, Methylparaben, Ethylparaben, Butylparaben, Propylparaben, Isobutylparaben | 0.80 |
| | Extrapon Kamille GW (Symrise) | Glycerin, Water (Aqua), Chamomilla Recutita (Matricaria) Flower Extract | 0.50 |
| | Extrapon Rosmarin GW (Symrise) | Glycerin, Water (Aqua), Rosmarinus officinalis (Rosemary) Leaf Extract | 0.30 |
| | Extrapon Grüner Tee GW (Symrise) | Glycerin, Water (Aqua), Camellia Sinensis Leaf Extract | 0.20 |
| | **Mädesüßkraut-Extrakt** | | **0.960** |
| | **Aloe Extrakt** | | **0.030** |
| | **Magnesium Ascorbylphosphat** | | **0.003** |
| | Propylenglycol-1,2 99P GC | Propylene Glycol | 5.00 |
| | Glycerin 85 P. | Glycerin | 2.00 |
| **C** | Natriumhydroxid (10% wässr. Lösung) | Sodium Hydroxide | 0.25 |
| **D** | Symrise Parfümöl | Fragrance | 0.30 |

Carbopol Ultrez-10 und Keltrol RD im Wasser vorquellen und die restlichen Rohstoffe von Teil B dazugeben. Teil A und B getrennt auf ca. 80°C erhitzen. Teil A zu Teil B geben und emulgieren, dabei Teil C zugeben. Kaltrühren und bei ca. 35°C Teil D zugeben. Der pH-Wert des Endprodukts sollte bei ca. 5,5 liegen.

4.7 Shampoo gegen Hautalterung

| Teil | Rohstoffname (Hersteller) | INCI-Bezeichnung | Gehalt in Gew.-% |
|---|---|---|---|
| **A** | Genapol LRO Flüssig (Cognis) | Sodium Laureth Sulfate | 37.00 |
| | Dragoderm (Symrise) | Glycerin, Triticum Vulgare (Wheat) Gluten, Water (Aqua) | 2.00 |
| | **Himbeerblatt-Extrakt** | | **0.01** |
| | **Aloe Extrakt** | | **0.9** |
| | **Magnesium Ascorbylphosphat** | | **0.09** |
| **B** | Demineralisiertes Wasser | Wasser (Aqua) | add 100 |
| | Merquat 550 (Ondeo Nalco) | Polyquaternium-7 | 0.50 |
| **C** | Demineralisiertes Wasser | Wasser (Aqua) | 20.00 |

(fortgesetzt)

| Teil | Rohstoffname (Hersteller) | INCI-Bezeichnung | Gehalt in Gew.-% |
|---|---|---|---|
| | Comperlan 100 (Cognis) | Cocamide MEA | 0.50 |
| D | Tego Betain L7 unkons. (Degussa-Goldschmidt) | Cocamidopropyl Betain | 6.00 |
| | Citronensäure 10%ig | Citric Acid | 0.30 |
| | EDETA B Pulver (BASF) | Tetrasodium EDTA | 0.10 |
| | Natriumbenzoat | Sodium Benzoate | 0.50 |
| | Natriumchlorid | Sodium Chloride | 1.00 |
| | Symrise Parfümöl | Fragrance | 0.50 |

Dragoderm und Brombeerblatt-Extrakt in Genapol LRO lösen. Merquat 550 in Wasser vorlösen und zugeben. Teil C unter Rühren und Erwärmen lösen und abkühlen lassen. Teil C in Teil A/B lösen. Die Rohstoffe von Teil D nacheinander zugeben und rühren. Der pH-Wert des Endprodukts sollte bei ca. 5,0 liegen.

4.8 Creme O/W gegen Hautalterung

| Teil | Rohstoffname (Hersteller) | INCI-Bezeichnung | Gehalt in Gew.-% |
|---|---|---|---|
| A | Dracorin CE (Symrise) | Glyceryl Stearate Citrate | 5.00 |
| | Neutralöl (Symrise) | Caprylic/Capric Triglyceride | 6.00 |
| | Isopropylpalmitat (Symrise) | Isopropyl Palmitate | 4.00 |
| | Lanette 16 (Cognis) | Cetyl Alcohol | 1.00 |
| | PCL Liquid 100 (Symrise) | Cetearyl Ethylhexanoate | 3.00 |
| | Dragoxat EH (Symrise) | Ethylhexyl Ethylhexanoate | 3.00 |
| | Abil 350 (Degussa-Goldschmidt) | Dimethicone | 0.50 |
| B | Demineralisiertes Wasser | Wasser (Aqua) | add 100 |
| | Keltrol RD (Rahn) | Xanthan Gum | 0.20 |
| | Symdiol 68 (Symrise) | 1,2 Hexanediol, Caprylylglycol | 0.50 |
| | Drago-Beta-Glucan (Symrise) | Water (Aqua), Butylene Gylcol, Glycerin, Avena Sativa (Oat) Kernel Extract | 0.30 |
| | **Brombeerblatt-Extrakt** | | **0.02** |
| | **Aloe Extrakt** | | **1.80** |
| | **Natrium Ascor-bylphosphat** | | **0.18** |
| | Glycerin 85 P. | Glycerin | 3.00 |
| C | Symrise Parfümöl | Fragrance | 0.30 |

Keltrol in Wasser quellen und die restlichen Rohstoffe von Teil B bis auf Drago-Beta-Glucan zugeben. Teil A und B getrennt auf ca. 80 °C erhitzen. Drago-Beta-Glucan zu Teil B geben. Teil B zu Teil A geben, danach erst emulgieren. Kaltrühren und bei ca. 35°C Teil C zugeben. Der pH-Wert des Endprodukts sollte bei ca. 5,5 liegen.

Beispiel 5: weitere Anwendungsbeispiele auf dem Gebiet der Mundhygiene

Zur Anwendung werden die erfindungsgemäßen, synergistisch wirksamen ternären Blattextrakt-enthaltenden Mundhygieneprodukte in Kombination mit anderen Wirk- und Zusatzstoffen in der üblichen Weise in die Mundhöhle in ausreichender Menge eingebracht. Nachfolgend sind beispielhaft vorteilhafte Zubereitungen für einige Anwendungen angegeben. Sofern nicht anders angegeben beziehen sich alle Angaben auf das Gewicht.

5.1. Gel-Zahncreme mit Wirksamkeit gegen Mundgeruch

| | I (%) | II (%) | III (%) |
|---|---|---|---|
| Na-carboxymethylcellulose | 0,40 | 0,40 | 0,40 |
| Sorbitol 70 %, in Wasser | 72,00 | 72,00 | 72,00 |
| Polyethylenglykol (PEG) 1500 | 3,00 | 3,00 | 3,00 |
| Na-saccharinat | 0,07 | 0,07 | 0,07 |
| Na-fluorid | 0,24 | 0,24 | 0,24 |
| p-Hydroxybenzoesäure (PHB)-Ethylester | 0,15 | 0,15 | 0,15 |
| Aroma A (siehe unten) | 0,10 | 0,80 | 0,75 |
| Ternäre Mischung aus Aloe Extrakt, Natrium Ascorbylphosphat und Himbeerblatt Extrakt (Mengenverhältnis: 90:9:1; w/w/w) | 0,01 | 0,40 | 1,00 |
| Abrasivkieselsäure | 11,00 | 11,00 | 11,00 |
| Verdickungskieselsäure | 6,00 | 6,00 | 6,00 |
| Natriumdodecylsulfat (SDS) | 1,40 | 1,40 | 1,40 |
| Wasser dest. | Ad 100,00 | Ad 100,00 | Ad 100,00 |

Aroma A hatte folgende Zusammensetzung:

30 Gew.-% l-Menthol, 30 Gew.-% Pfefferminzöl Mentha piperita, 21,5 Gew.-% Pfefferminzöl Mentha arvensis, 9 Gew.-% Anethol, 0,5 Gew.-% Anisaldehyd, 2 Gew.-% Eucalyptol, 1 Gew.-% Eucalyptus globulus Öl, 3 Gew.-% Menthon, 1 Gew.-% Spearmintöl, 1 Gew.-% Basilikumöl, 0,5 Gew.-% Menthylacetat, 0,05 Gew.-% Menthyllactat, 0,1 Gew.-% Menthyl-3-carbonsäure-N-ethylamid (WS-3), 0,05 Gew.-% 2-Hydroxyethylmenthylcarbonat (Frescolat MGC, Symrise), 0,05 Gew.-% 2-Hydroxypropylmenthylcarbonat (Frescolat MPC, Symrise), 0,1 Gew.-% Pinen, 0,1 Gew.-% Propylenglykol, 0,05 Gew.-% Limonen.

5.2. Zahncreme mit Wirksamkeit gegen Plaque

| | I (%) | II (%) | III (%) |
|---|---|---|---|
| Na-carboxymethylcellulose | 1,00 | 1,00 | 1,00 |
| Glycerin | 12,50 | 12,50 | 12,50 |
| Sorbitol 70 %, in Wasser | 29,00 | 29,00 | 29,00 |
| Na-saccharinat | 0,20 | 0,20 | 0,20 |
| Na-fluorid | 0,22 | 0,22 | 0,22 |
| Azacycloheptan-2,2-diphosphosäure, Di-natriumsalz | 1,00 | 1,00 | 1,00 |
| Bromchlorophen | 0,10 | 0,10 | 0,10 |

(fortgesetzt)

| | I (%) | II (%) | III (%) |
|---|---|---|---|
| Pfefferminz-Aroma | 0,05 | 1,10 | 0,35 |
| Ternäre Mischung aus Aloe Extrakt, Magnesium Ascorbylphosphat und Himbeerblatt Extrakt (Mengenverhältnis: 90:9:1; w/w/w) | 1,05 | 1,30 | 1,70 |
| Kamillen Extrakt (Cremogen Forte Kamille, Symrise) | 0,20 | 0,50 | 0,10 |
| Salbei Extrakt (Extrapon Salbei GW, Symrise) | 0, 20 | 0,10 | 0,30 |
| Abrasivkieselsäure | 14,00 | 14,00 | 14,00 |
| Verdickungskieselsäure | 5,00 | 5,00 | 5,00 |
| Natriumdodecylsulfat (SDS) | 1,50 | 1,50 | 1,50 |
| Wasser dest. | Ad 100,00 | Ad 100,00 | Ad 100,00 |

5.3. Zahncreme mit Wirksamkeit gegen Plaque

Basis: Kieselsäure, Alkalidiphosphat

| | I (%) | II (%) | III (%) |
|---|---|---|---|
| Carragenan | 0,90 | 0,90 | 0,90 |
| Glycerin | 15,00 | 15,00 | 15,00 |
| Sorbitol 70 %, in Wasser | 25,00 | 25,00 | 25,00 |
| PEG 1000 | 3,00 | 3,00 | 3,00 |
| Na-fluorid | 0,24 | 0,24 | 0,24 |
| Tetrakalium-diphosphat | 4,50 | 4,50 | 4,50 |
| Tetranatrium-diphosphat | 1,50 | 1,50 | 1,50 |
| Na-saccharinat | 0,40 | 0,40 | 0,40 |
| Fällungskieselsäure | 20,00 | 20,00 | 20,00 |
| Titandioxid | 1,00 | 1,00 | 1,00 |
| PHB-Methylester | 0,10 | 0,10 | 0,10 |
| Menthol-Eucalyptol - Aroma | 1,10 | 0,80 | 0,20 |
| Ternäre Mischung aus Aloe Extrakt, Natrium Ascorbylphosphat und Brombeer-blatt Extrakt (Mengenverhältnis: 90:9:1; w/w/w) | 0,10 | 0,40 | 1,00 |
| Natriumdodecylsulfat | 1,30 | 1,30 | 1,30 |
| Wasser dest. | Ad 100,00 | Ad 100,00 | Ad 100,00 |

5.4. Zahncreme gegen empfindliche Zähne

| | I (%) | II (%) | III (%) |
|---|---|---|---|
| Na-carboxymethylcellulose | 0,70 | 0,70 | 0,70 |
| Xanthan Gum | 0,50 | 0,50 | 0,50 |
| Glycerin | 15,00 | 15,00 | 15,00 |
| Sorbitol 70 %, in Wasser | 12,00 | 12,00 | 12,00 |
| K-nitrat | 5,00 | 5,00 | 5,00 |
| Na-monofluorphosphat | 0,80 | 0,80 | 0,80 |
| PHB-Methylester | 0,15 | 0,15 | 0,15 |
| PHB-Propylester | 0,05 | 0,05 | 0,05 |
| Na-saccharinat | 0,20 | 0,20 | 0,20 |
| Menthol-Anethol - Aroma | 0,25 | 0,75 | 0,25 |
| Ternäre Mischung aus Aloe Extrakt, Natrium Ascorbylphosphat und Himbeerblatt Extrakt (Mengenverhältnis: 90:9:1; w/w/w) | 0,02 | 1,00 | 1,50 |
| Rosmarin-Extrakt (Extrapon Rosamrin, Symrise) | 0,20 | 0,01 | 0,10 |
| Ca-carbonat | 35,00 | 35,00 | 35,00 |
| Siliciumdioxid | 1,00 | 1,00 | 1,00 |
| Natriumdodecylsulfat (SDS) | 1,50 | 1,50 | 1,50 |
| Wasser dest. | Ad 100,00 | Ad 100,00 | Ad 100,00 |

5.5. Zahncreme gegen empfindliche Zähne

| | I (%) | II (%) | III (%) |
|---|---|---|---|
| Hydroxyethylcellulose | 1,40 | 1,40 | 1,40 |
| Guar Gum | 0,60 | 0,60 | 0,60 |
| Glycerin | 18,00 | 18,00 | 18,00 |
| Sorbitol 70 %, in Wasser | 12,00 | 12,00 | 12,00 |
| Na-Saccharinat | 0,35 | 0,35 | 0,35 |
| Farbstoff | 0,01 | 0,01 | 0,01 |
| PHB-Methylester | 0,15 | 0,15 | 0,15 |
| PHB-Propylester | 0,04 | 0,04 | 0,04 |
| Sr-chlorid | 10,50 | 10,50 | 10,50 |
| Pfefferminz-Anis-Aroma | 0,35 | 1,20 | 0,60 |
| Ternäre Mischung aus Aloe Extrakt, Magnesium Ascorbylphosphat und Brombeerblatt Extrakt (Mengenverhältnis: 90:9:1; w/w/w) | 0,05 | 0,50 | 0,90 |

(fortgesetzt)

| | I (%) | II (%) | III (%) |
|---|---|---|---|
| Grüntee-Extrakt (Extrapon Grüntee GW, Symrise | 0,25 | 0,10 | 0,05 |
| Fällungskieselsäure | 15,00 | 15,00 | 15,00 |
| Siliciumdioxid | 1,60 | 1,60 | 1,60 |
| Natriumdodecylsulfat | 1,30 | 1,30 | 1,30 |
| Wasser dest. | Ad 100,00 | Ad 100,00 | Ad 100,00 |

5.6. Gebrauchsfertiges Mundwasser mit Fluorid

| | I (%) | II (%) | III (%) |
|---|---|---|---|
| Ethanol | 7,00 | 7,00 | 7,00 |
| Glycerin | 12,00 | 12,00 | 12,00 |
| Na-fluorid | 0,05 | 0,05 | 0,05 |
| Pluronic F-127® (BASF, oberflächenaktive Substanz) | 1,40 | 1,40 | 1,40 |
| Na-phosphatpuffer pH 7,0 | 1,10 | 1,10 | 1,10 |
| Sorbinsäure | 0,20 | 0,20 | 0,20 |
| Na-saccharinat | 0,10 | 0,10 | 0,10 |
| Menthol-Pfefferminz-Aroma | 0,08 | 0,20 | 0,15 |
| Ternäre Mischung aus Aloe Extrakt, Magnesium Ascorbylphosphat und Erdbeerblatt Extrakt (Mengenverhältnis: 96.5 : 3.2 : 0.3 w/w/w) | 0,02 | 0,70 | 0,10 |
| | | | |
| Bisabolol | 0,01 | 0,05 | 0,20 |
| Melissen-Extrakt (Extrapon Melisse, Symrise) | 0,30 | 0,50 | 0,05 |
| Salbei Extrakt (Extrapon Salbei, Symrise) | 0,30 | 0,10 | 0,05 |
| Farbstoff | 0,01 | 0,01 | 0,01 |
| Wasser dest. | Ad 100,00 | Ad 100,00 | Ad 100,00 |

5.7. Mundwasserkonzentrat

| | I (%) | II (%) | III (%) |
|---|---|---|---|
| Ethanol, 95%ig | 80,00 | 80,00 | 80,00 |
| Na-cyclamat | 0,15 | 0,15 | 0,15 |
| Menthol-Anis-Eucalyptol - Aroma | 1,50 | 2,00 | 2,00 |
| Farbstoff | 0,01 | 0,01 | 0,01 |

(fortgesetzt)

| | I (%) | II (%) | III (%) |
|---|---|---|---|
| Ternäre Mischung aus Aloe Extrakt, Natrium Ascorbylphosphat und Mädesüßkraut Extrakt (Mengenverhältnis: 90:9:1; w/w/w) | 1,50 | 2,50 | 5,00 |
| Grüntee-Extrakt (Extrapon Grüntee GW, Symrise) | 0,20 | 1,00 | 0,50 |
| Bisabolol | 0,50 | 0,20 | 1,00 |
| Wasser dest. | Ad 100,00 | Ad 100,00 | Ad 100,00 |

5.8. Kaugummi

| | I (%) | II (%) | III (%) |
|---|---|---|---|
| Kaugummibase | 21,00 | 21,00 | 21,00 |
| Glucose Sirup | 16,50 | 16,50 | 16,50 |
| Glycerin | 0,50 | 0,50 | 0,50 |
| Puderzucker | 60,30 | 60,00 | 59,80 |
| Menthol-Spearmint-Aroma | 1,20 | 1,00 | 0,70 |
| Ternäre Mischung aus Aloe Extrakt, Magnesium Ascorbylphosphat und Erdbeerblatt Extrakt (Mengenverhältnis: 90:9:1; w/w/w) | 0,50 | 1,00 | 1,50 |

5.9. Zuckerfreier Kaugummi

| | I (%) | II (%) | III (%) |
|---|---|---|---|
| Kaugummibase | 30,00 | 30,00 | 30,00 |
| Sorbit, Pulver | 38,45 | 38,40 | 38,30 |
| Palatinit | 9,50 | 9,50 | 9,50 |
| Xylit | 2,00 | 2,00 | 2,00 |
| Mannit | 3,00 | 3,00 | 3,00 |
| Aspartame | 0,10 | 0,10 | 0,10 |
| Acesulfam K | 0,10 | 0,10 | 0,10 |
| Emulgum / Emulgator | 0,30 | 0,30 | 0,30 |
| Sorbitol 70 %, in Wasser | 14,00 | 14,00 | 14,00 |
| Glycerin | 1,00 | 1,00 | 1,00 |

(fortgesetzt)

| | I (%) | II (%) | III (%) |
|---|---|---|---|
| Menthol-Anis-Zimt-Aroma | 1,20 | 0,80 | 0,60 |
| Ternäre Mischung aus Aloe Extrakt, Natrium Ascorbylphosphat und Himbeerblatt Extrakt (Mengenverhältnis: 96.5 : 3.2 : 0.3; w/w/w) | 0,35 | 0,80 | 1,10 |

5.10. Gelatinekapsel zum Direktverzehr

| | I (%) | II (%) | III (%) |
|---|---|---|---|
| Gelatinehülle: | | | |
| Glycerin | 2,014 | 2,014 | 2,014 |
| Gelatine 240 Bloom | 7,91 | 7,91 | 7,91 |
| Sucralose | 0,065 | 0,065 | 0,065 |
| Allura Rot | 0,006 | 0,006 | 0,006 |
| Brillantblau | 0,005 | 0,005 | 0,005 |
| | | | |
| Kernzusammensetzung: | | | |
| Pflanzenöl-Triglycerid (Kokosöl - Fraktion) | 80,0 | 75,0 | 77,5 |
| Aroma B | 9,95 | 12,0 | 12,0 |
| Ternäre Mischung aus Aloe Extrakt, Magnesium Ascorbylphosphat und Himbeerblatt Extrakt (Mengenverhältnis: 90:9:1; w/w/w) | 0,05 | 3,0 | 0,5 |

Aroma B hatte dabei folgende Zusammensetzung (Angaben jeweils in Gew.-%):

0,1% Neotam Pulver, 0,05% Aspartam, 29,3% Pfefferminzöl arvensis, 29,3% Pfefferminz piperita Öl Willamette, 2,97% Sucralose, 2,28% Triacetin, 5,4% Diethyltartrat, 12,1% Pfefferminzöl yakima, 0,7% Ethanol, 3,36% 2-Hydroxyethylmenthylcarbonat, 3,0% 2-Hydroxypropylmenthylcarbonat, 0,27% Vanillin, 5,5% D-Limonen, 5,67% L-Menthylacetat.

Die zum Direktverzehr geeignete Gelatinekapsel (Herstellung analog zu WO 2004/050069) hatte einen Durchmesser von 5 mm, das Gewichtsverhältnis von Kernmaterial zu Hüllmaterial lag bei 90 : 10. Die Kapsel öffnete sich im Mund innerhalb von weniger als 10 Sekunden und löste sich vollständig innerhalb von weniger als 50 Sekunden auf.

**Beispiele 6:** Weitere Formulierungen umfassend erfindungsgemäße Zubereitungen mit Collagensynthese stimulierender Wirksamkeit

In der nachfolgenden Tabelle bedeuten

**6.1 = Hautaufhellende Tagescreme O/W**
**6.2 = Hautberuhigende Lotion mit Pflanzenextrakten O/W**
**6.3 = After Sun Balm**
**6.4 = Körperspray**
**6.5 = Sonnenschutzlotion (O/W), Breitbandschutz**

6.6 = W/O Nachtcreme
6.7 = Shampoo
6.8 = Selbstbräunungscreme
6.9 = Barrierereparaturcreme O/W
6.10 = Antitranspirant/Deodorant Roll-on

| ROHMATERIAL-NAME (HERSTELLER) | INCI | GEWICHTS % | | | | | | | | | |
|---|---|---|---|---|---|---|---|---|---|---|---|
| | | 6.1 | 6.2 | 6.3 | 6.4 | 6.5 | 6.6 | 6.7 | 6.8 | 6.9 | 6.10 |
| Aloe Extrakt | | 0.9 | 1.8 | 0.9 | 2.0 | 0.8 | 1.5 | 2.7 | 2.0 | 0.6 | 1.0 |
| Natrium Ascorbylphos-phat | | 0.09 | | 0.09 | 0.5 | | 0.15 | | 0.5 | | 0.2 |
| Magnesium Ascorbylphos-phat | | | 0.18 | | | 0.3 | | 0.27 | | 0.06 | |
| Himbeerblatt Extrakt | | 0.01 | | | 0.1 | 0.1 | 0.05 | | | | 0.02 |

| ROHMATERIAL-NAME (HERSTELLER) | INCI | GEWICHTS % | | | | | | | | | |
|---|---|---|---|---|---|---|---|---|---|---|---|
| | | 6.1 | 6.2 | 6.3 | 6.4 | 6.5 | 6.6 | 6.7 | 6.8 | 6.9 | 6.10 |
| **Mädesüßkrautextrakt** | | | **0.02** | | | | | | **0.1** | | |
| **Erbeerblatt Extrakt** | | | | | | | | **0.03** | | **0.01** | |
| **Brombeerblatt Extrakt** | | | | **0.01** | | | | | | | |
| -(-Alpha-)-Bisabolol, natural (Symrise) | Bisabolol | 0.3 | 0.4 | 0.3 | 0.1 | 0.3 | 0.2 | 0.05 | 0.2 | 0.5 | 0.1 |
| Ingwer $CO_2$ Extrakt (Flavex) | Zingiber Officinale (Ginger) Root Extract | 0.003 | 0.004 | 0.003 | 0.005 | 0.003 | 0.002 | 0.001 | 0.002 | 0.01 | 0.001 |
| Abil 350 (Degussa-Goldschmidt) | Dimethicone | 0.5 | 2.0 | 1.0 | | | | | 0.5 | 0.5 | |
| Allantoin (Merck) | Allantoin | | 0.2 | 0.1 | | | | | | | |
| Aloe Vera Gel Concentrate 10/1 (Symrise) | Water (Aqua), Aloe Barbadensis Leaf Juice | | | 3.0 | | | 3.0 | | | | |
| Alugel 34 TH (Baerlocher) | Aluminium Stearate | | | | | | 1.0 | | | | |
| Aqua-Ceramide (Kao) | Cetyloxypropyl Glyceryl Methoxypropyl Myristamide | | 0.1 | | | | | | | | 0.1 |
| Arbutin (Sabinsa) | ß-Arbutin | 1.0 | | | | | | | | | |
| Natrium Ascorbyl Phosphate (EMD Chemicals) | Natrium Ascorbyl Phosphate | 2.0 | | 1.0 | | | | | | | |
| Butylene Glycol | Butylene Glycol | | | 5.0 | | | | | | | |
| Carbopol ETD 2050 (Noveon) | Carbomer | | | | | 0.2 | | | | | |
| Carbopol Ultrez-10 (Noveon) | Carbomer | | 0.1 | | | | | | | | |
| Ceramide 2 (Sederma) | Ceramide 2 | 0.1 | | | | | | | | | |

| ROHMATERIAL-NAME (HERSTELLER) | INCI | GEWICHTS % | | | | | | | | | |
|---|---|---|---|---|---|---|---|---|---|---|---|
| | | 6.1 | 6.2 | 6.3 | 6.4 | 6.5 | 6.6 | 6.7 | 6.8 | 6.9 | 6.10 |
| Ceramide PC104 (Pacific Corporation) | Hydroxypropyl Bispalmitamide MEA | | | | | 0.1 | | | | | |
| Ceramide SL (Sino Lion) | Hydroxyethyl Palmityl O-xyhydroxypropyl Palmitamide | | | | | | 0.1 | | | | |
| Cetiol OE (Cognis) | Dicaprylyl Ether | | | 4.0 | | | | | | | |
| Cetiol SB 45 (Cognis) | Butyrospermum Parkii (Shea Butter) | | | 1.0 | | | | | | | |
| Citric Acid 10% sol. | Citric Acid | | | | | | | 0.3 | | | |
| Comperlan 100 (Cognis) | Cocamide MEA | | | | | | | 0.5 | | | |
| Dihydroxyace-ton (Merck) | Dihydroxyacetone | | | | | | | | 5.0 | | |
| Dow Corning 246 Fluid (Dow Corning) | Cyclohexasilox-ane and Cyclopentasilox-ane | | | | | 2.0 | | | | | |
| Dow Corning 345 Fluid (Dow Corning) | Cyclomethicone | | | | 0.5 | | | | | | |
| D-Panthenol (BASF) | Panthenol | | | 1.0 | | | | | | | |
| Dracorin CE (Symrise) | Glyceryl Stearate Citrate | 5.0 | | | | | | | 5.0 | 1.5 | |
| Dracorin GMS (Symrise) | Glyceryl Stearate | | 2.0 | | | | | | | 2.0 | |
| Dracorin GOC (Symrise) | Glyceryl Oleate Citrate, Caprylic/Capric Triglyceride | | | | 2.0 | | | | | | |
| Drago-Beta-Glucan (Sym-rise) | Water (Aqua), Butylene Glycol, Glycerin, Avena Sativa (Oat), Kernel Extract | 0.3 | | | | | | | | | |

| ROHMATERIAL-NAME (HERSTELLER) | INCI | GEWICHTS % | | | | | | | | | |
|---|---|---|---|---|---|---|---|---|---|---|---|
| | | 6.1 | 6.2 | 6.3 | 6.4 | 6.5 | 6.6 | 6.7 | 6.8 | 6.9 | 6.10 |
| Dragocid Liquid (Symrise) | Phenoxyethanol, Methylparaben, Ethylparaben, Butylparaben, Propylparaben, Isobutylparaben | | 0.8 | 0.7 | | 0.7 | 0.8 | | | 0.8 | |
| Dragoderm (Symrise) | Glycerin, Triticum Vulgare (Wheat) Gluten, Water (Aqua) | | | | | | | 2.0 | | | |
| Drago-Oat-Active (Symrise) | Water (Aqua), Butylene Gylcol, Avena Sativa (Oat) Kernel Extract | | | | 1.0 | | | | | | |
| Dragosan W/O Liquid (Symrise) | Polyglyceryl-3-Polyricinoleate, Sorbitan Isostearate | | | | | | 1.0 | | | | |
| Dragosan W/O P (Symrise) | Sorbitan Isostearate, Hydrogenated Castor Oil, Ceresin, Beeswax (Cera Alba) | | | | | | 6.0 | | | | |
| Dragoxat EH (Symrise) | Ethylhexy Ethylhexanoate | 3.0 | 3.0 | | 4.0 | | | | 3.0 | | |
| Dragoxat 89 (Symrise) | Ethylhexyl Ethylisonona-noate | | | | | | | | | 2.0 | |
| EDETA B Pulver (BASF) | Tetrasodium EDTA | | | | | | | 0.1 | | | |
| EDETA DB (BASF) | Disodium EDTA | | | | | 0.1 | | | 0,1 | | |
| Emulsiphos (Symrise) | Potassium Cetyl Phosphate, Hydrogenated Palm Glycerides | | 2.0 | | | 1.5 | | | | 2.0 | |
| Ethanol 96 % | Ethanol | | | | | | | | 2.0 | | 30.0 |

| ROHMATERIAL-NAME (HERSTELLER) | INCI | GEWICHTS % | | | | | | | | | |
|---|---|---|---|---|---|---|---|---|---|---|---|
| | | 6.1 | 6.2 | 6.3 | 6.4 | 6.5 | 6.6 | 6.7 | 6.8 | 6.9 | 6.10 |
| Extrapone Green Tea GW (Symrise) | Glycerin, Water (Aqua), Camellia Sinensis Leaf Extract | | 0.2 | | | | | | | | |
| Extrapone Witch Hazel Distillate colorless (Symrise) | Propylene Glycol, Hamamelis Virginiana (Witch Hazel) Water, Water (Aqua), Hamamelis Virginiana (Witch Hazel) Extract | | | | | | 1.0 | | | | |
| Extrapone Rosemary GW (Symrise) | Glycerin, Water (Aqua), Rosmarinus officinalis (Rosemary) Leaf Extract | | 0.3 | | | | | | | 0.5 | |
| Farnesol (Symrise) | Farnesol | | | | | | | | | | 0.5 |
| Frescolat ML crist. (Symrise) | Menthyl Lactate | | | 0.8 | | | | | | | |
| Genapol LRO liquid (Cognis) | Sodium Laureth Sulfate | | | | | | | 37.0 | | | |
| Givobio GZN (Seppic) | Zinc Gluconate | | | | | | | | | 0.5 | |
| Glycerin 85 % | Glycerin | 3.0 | 2.0 | 4.0 | | 4.7 | 2.0 | | 1.5 | 3.0 | |
| Hydrolite-5 (Symrise) | Pentylene Glycol | | | | 5.0 | | | | 3.5 | | |
| Hydroviton (Symrise) | Water, Glycerin, Sodium Lactate, TEA Lactate, Serine, Lactic Acid, Urea, Sorbitol, Sodium Chloride, Lauryl Diethylenediaminoglycine, Lauryl Aminopropylglycine, Allantoin | | | | | | | | | 1.0 | |

| ROHMATERIAL-NAME (HERSTELLER) | INCI | GEWICHTS % | | | | | | | | | |
|---|---|---|---|---|---|---|---|---|---|---|---|
| | | 6.1 | 6.2 | 6.3 | 6.4 | 6.5 | 6.6 | 6.7 | 6.8 | 6.9 | 6.10 |
| Irgasan DP 300 (Ciba Geigy) | Triclosan | | | | | | | | | | 0.3 |
| Isodragol (Symrise) | Triisononanoin | | 2.0 | | | | | | | 3.0 | |
| Isopropylpalmitat (Symrise) | Isopropyl Palmitate | 4.0 | | | | | | | 4.0 | | |
| Karion F (Merck) | Sorbitol | | | | | | 2.0 | | | | |
| Keltrol RD (CP-Kelco) | Xanthan Gum | 0.2 | 0.1 | | | | | | | | |
| Keltrol T (Danby-Chemie) | Xanthan Gum | | | | | 0.2 | | | 0.3 | | |
| Kojic acid (Cosmetochem) | Kojoc acid | 1.0 | | | | | | | | | |
| Lanette 16 (Cognis) | Cetyl Alcohol | 1.0 | | | | | | | 1.0 | | |
| Lanette O (Cognis) | Cetearyl Alcohol | | 3.0 | | | 1.0 | | | | 2.0 | |
| Lara Care A-200 (Rahn) | Galactoarabinan | | | 0.3 | | | | | | | |
| Magnesium Chloride (Merck) | Magnesium Chloride | | | | | | 0.7 | | | | |
| Merquat 550 (Ondeo Nalco) | Polyquaternium-7 | | | | | | | 0.5 | | | |
| NAOH 10% sol. | Sodium Hydroxide | | | | | | | | | 0.3 | |
| Naringin (Exquim) | 4',5,7-Trihydroxyflavon-7-O-neohesperidosid | | | | | | | 0.5 | 2.0 | | |
| Natriumbenzoat | Sodium Benzoate | | | | | | | 0.5 | | | |
| Natrosol 250 HHR (Aqualon) | Hydroxyethylcellulose | | | | | | | | | | 0.3 |
| Neo Heliopan 357 (Symrise) | Butyl Methoxydibenzoylmethane | | | | | 1.0 | | | | | |

| ROHMATERIAL-NAME (HERSTELLER) | INCI | GEWICHTS % | | | | | | | | | |
|---|---|---|---|---|---|---|---|---|---|---|---|
| | | 6.1 | 6.2 | 6.3 | 6.4 | 6.5 | 6.6 | 6.7 | 6.8 | 6.9 | 6.10 |
| Neo Heliopan AP (Symrise) (10 % als Natriumsalz) | Disodium Phenyl Dibenzimidazole Tetrasulfonate | | | | | 10 | | | | | |
| Neo Heliopan AV (Symrise) | Ethylhexyl Methoxycinnamate | | | | | 3.0 | | | | | |
| Neo Heliopan Hydro (Symrise) (15 % als Natriumsalz) | Phenylbenzimidazole Sulfonic Acid | | | | | 6.7 | | | | | |
| Neo Heliopan MBC (Symrise) | 4-Methylbenzylidene Camphor | | | | | 1.5 | | | | | |
| Neo Heliopan OS (Symrise) | Ethylhexyl Salicylate | | | | | 5.0 | | | | | |
| Neutral Oil | Caprylic/Capric Triglyceride | 6.0 | | | 4.0 | 2.0 | | | 6.0 | 10.0 | |
| Oxynex 2004 (Merck) | BHT | | | | | | 0.1 | | | | |
| Paraffinöl 5 Grad E (Parafluid) | Paraffinum Liquidum | | | | 4.0 | | | | | | |
| PCL Liquid 100 (Symrise) | Cetearyl Ethylhexoate | 3.0 | 5.0 | | 7.0 | | | | | | |
| PCL Solid (Symrise) | Stearyl Heptanoate, Stearyl Caprylate | | 2.0 | | | | | | | | |
| PCL-Liquid (Symrise) | Cetearyl Ethylhexanoate, Isopropyl Myristate | | | | | | 12.0 | | 3.0 | | |
| Pemulen TR-2 (Noveon) | Acrylates/C10-30 Alkyl Acrylate Crosspolymer | | | 0.3 | 0.2 | | | | | | |
| 4-(1-Phenylethyl)1,3-benzenediol | 4-(1-Phenylethyl)1,3-benzenediol | 0.5 | | | | | | | | | |
| Propylene Glycol-1,2 99P GC | Propylene Glycol | | 5.0 | | | | | | | | |

| ROHMATERIAL-NAME (HERSTELLER) | INCI | GEWICHTS % | | | | | | | | | |
|---|---|---|---|---|---|---|---|---|---|---|---|
| | | 6.1 | 6.2 | 6.3 | 6.4 | 6.5 | 6.6 | 6.7 | 6.8 | 6.9 | 6.10 |
| Pseudoceramide 391 | N-(1-Hexadecanoyl)-4-hydroxy-L-prolin-(1-hexadecyl -ester | | 0.1 | | | | | 0.2 | | 0.5 | |
| Retinyl Palmitate in Oil (DSM Nutrional Products) | Retinyl Palmitate | | | | | | 0.2 | | | | |
| Sepigel 305 | Polyacrylamide, C13-14 Isoparaffin, Laureth-7 | | | | | | | | 1.0 | | |
| Sodium Chloride | Sodium Chloride | | | | | | | 1.0 | | | |
| Sodium Hydroxide (10% sol. | Sodium Hydroxide | | 0.3 | 0.6 | 0.4 | | | | | | |
| Solubilizer 611674 (Symrise) | PEG-40 Hydrogenated Castor Oil, Trideceth-9, Water (Aqua) | | | | | | | | | | 2.0 |
| Sun Flower Oil (Wagner) | Helianthus Annuus (Sunflower) Seed Oil | | | | | | 5.0 | | | | |
| Sweet Almond Oil (Wagner) | Prunus dulcis | | | | | | 5.0 | | | | |
| Symdiol 68 (Symrise) | 1,2 Hexanediol, Caprylylglycol | 0.5 | | | | | | | | | |
| Symrise Fragrance | Fragrance | 0.3 | 0.3 | 0.3 | 0.2 | 0.4 | 0.4 | 0.5 | 0.3 | 0.3 | 1.0 |
| Tamasterol (Tama Biochemicals) | Phytosterols | | | | | | | | | 0.3 | |
| Tego Betain L7 (Degussa) | Cocamidopropyl Betain | | | | | | | 6.0 | | | |
| Tegosoft PC 31(Degussa) | | | | | | | | | | 0.3 | |
| Tegosoft TN (Degussa) | C12-15 Alkyl Benzoate | | | 5.0 | | 5.0 | | | | | |
| Triethanolamine, 99% | Triethanolamine | | | | | 0.5 | | | | | |

| ROHMATERIAL-NAME (HERSTELLER) | INCI | GEWICHTS % | | | | | | | | | |
|---|---|---|---|---|---|---|---|---|---|---|---|
| | | 6.1 | 6.2 | 6.3 | 6.4 | 6.5 | 6.6 | 6.7 | 6.8 | 6.9 | 6.10 |
| Tocopherol Acetate (DSM Nutritional Products) | Tocopheryl Acetate | | | 0.5 | | 0.5 | 3.0 | | | 0.3 | |
| Zirkonal L 450 (BK Giulini) | Aluminium Zirconium Pentachloro-hydrate (40%ige wässrige Lösung) | | | | | | | | | | 37.0 |
| Water, demin-eralized | Wasser (Aqua) | ad 100 | ad 100 | ad 100 | ad 100 | ad 100 | ad 100 | ad 100 | ad 100 | ad 100 | ad 100 |

## Patentansprüche

**1.** Ternäre Mischung enthaltend, bestehend im wesentlichen aus oder bestehend aus

a) einem Extrakt aus Aloe-Pflanzen-Material,
b) Ascorbinsäure und/oder deren Derivat(e), vorzugsweise zumindest einem Solvat und/oder Salz der Ascorbinsäure und/oder zumindest einem Ascorbinsäure-Prodrug, und
c) einem Extrakt aus Rosaceae-Pflanzen-Material,

ausgenommen einem After-sun-Balsam ("after-sun balm") enthaltend gleichzeitig 3.0 Gew.-% an Aloe barbadensis-Blattextrakt 10/1 in Wasser, 1.0 Gew.-% Natrium-ascorbylphosphat und 0.5 Gew.-% Maltodextrin-Rubus fruticosus-Blattextrakt, jeweils bezogen auf den gesamten Balsam.

**2.** Mischung nach Anspruch 1, enthaltend

a) einen Extrakt aus Aloe-Pflanzen-Material,
b) Natrium- und/oder Magnesium-Ascorbylphosphat, und
c) einem Extrakt ausgewählt aus der Gruppe bestehend aus Brombeerblattextrakt, Himbeerblattextrakt, Erdbeerblattextrakt und Mädesüßkrautextrakt, oder zwei oder mehr solcher Extrakte.

**3.** Mischung nach Anspruch 1 oder 2, wobei die Anteile der Komponenten jeweils betragen:

Komponente a): 33-99 Gew.-%, vorzugsweise 80-98 Gew.-% und besonders bevorzugt 89-96,51 Gew.-%;
Komponente b): 0,99-32,99 Gew.-%, vorzugsweise 1,9-15 Gew.-% und besonders bevorzugt 3,22-9 Gew.-%;
Komponente c): 0,01-10 Gew.-%, vorzugsweise 0,1-4 Gew.-% und besonders bevorzugt 0,27-2 Gew.-%; jeweils bezogen auf die Summe der Komponenten a), b) und c).

**4.** Mischung nach Anspruch 1, 2 oder 3, wobei die Anteile der Komponente jeweils betragen:

Komponente a): 85-92 Gew.-%, vorzugsweise 88-92 Gew.-%, besonders bevorzugt 90 Gew.-%,
Komponente b): 7-10 Gew.-%, vorzugsweise 9 Gew.-%,
Komponente c): 0,5-2 Gew.-%, vorzugsweise 0,8-1,2 Gew.-%, besonders bevorzugt 1 Gew.-%, jeweils bezogen auf die Summe der Komponenten a), b) und c), wobei Komponente b) aus Magnesium-Ascorbylphosphat und Komponente c) aus Himbeerblatt-Extrakt besteht.

**5.** Kosmetische, pharmazeutische und/oder mundhygienische Zubereitung, enthaltend eine Mischung nach einem der Ansprüche 1, 2, 3 oder 4, wobei die Konzentration der Mischung 0,001 - 20 Gew.-% beträgt, vorzugsweise 0,01 - 10 Gew.-% und besonders bevorzugt 0,1 - 5 Gew.-% beträgt, bezogen auf die gesamte Zubereitung.

**6.** Kosmetische, pharmazeutische und/oder mundhygienische Zubereitung nach Anspruch 5, enthaltend die Mischung nach einem der Ansprüche 1, 2, 3 oder 4 in einer

- zum Stimulieren der Collagensynthese und/oder
- zum Verlangsamen der Hautalterung und/oder
- zum Vorbeugen und/oder Verlangsamen von Parodontitis und/oder Karies

ausreichenden Konzentration.

**7.** Verfahren zum Herstellen einer pulverförmigen Mischung nach Anspruch 1, 2, 3 oder 4, umfassend das Mischen der Komponenten a), b) und c) in ihren jeweils gewählten Mengen zum Erhalten einer homogenen pulverförmigen Mischung.

**8.** Verfahren zum Herstellen einer flüssigen Mischung nach Anspruch 1, 2, 3 oder 4, umfassend

(i) das Mischen der Komponenten a), b) und c) in ihren jeweils gewählten Mengen zum Erhalten einer, vorzugsweise homogenen, pulverförmigen Mischung, und anschließendes
(ii) Lösen der so erhaltenen pulverförmigen Mischung in einem Medium enthaltend

- Wasser und/oder
- einen Alkohol ausgewählt aus der Gruppe bestehend aus Methanol, Ethanol, n-Propanol, Isopropanol, und/oder
- ein aliphatisches Polyol ausgewählt aus der Gruppe bestehend aus Glycerin, Propylenglycol, Butylenglycol, Alkan-1,2-diole mit 3-10 Kohlenstoffatomen.

**9.** Verwendung einer Mischung nach einem der Ansprüche 1, 2, 3 oder 4 oder einer kosmetischen, pharmazeutischen und/oder mundhygienischen Zubereitung nach Anspruch 5 oder 6

- zum nicht-therapeutischen Stimulieren der Collagensynthese und/oder
- zum nicht-therapeutischen Verlangsamen der Hautalterung und/oder
- zum nicht-therapeutischen Vorbeugen und/oder Verlangsamen von Parodontitis und/oder Karies.

Europäisches Patentamt

# EUROPÄISCHER RECHERCHENBERICHT

Nummer der Anmeldung
EP 08 15 2815

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| D,Y | WO 2005/123101 A (SYMRISE GMBH & CO KG ET AL.) 29. Dezember 2005 (2005-12-29)<br>* Seite 26 - Seite 28; Beispiele *<br>* Seite 1, Absatz 1; Ansprüche *<br>----- | 1-9 | INV.<br>A61K8/97<br>A61Q19/08<br>A61K36/73 |
| Y | WO 2007/063087 A (SYMRISE GMBH & CO KG ET AL.) 7. Juni 2007 (2007-06-07)<br>* Seiten ,7,31; Ansprüche; Beispiele 1,3 *<br>----- | 1-9 | |
| A | WO 2006/015675 A (SYMRISE GMBH & CO KG ET AL.) 16. Februar 2006 (2006-02-16)<br>* Seite 7, Absatz 4 *<br>* Seite 9, Absatz 3-5 *<br>* Seiten 26,27; Ansprüche 1,4,14 *<br>----- | 1-9 | |

RECHERCHIERTE SACHGEBIETE (IPC)
A61K
A61Q

Der vorliegende Recherchenbericht wurde für alle Patentansprüche erstellt

2

| Recherchenort | Abschlußdatum der Recherche | Prüfer |
|---|---|---|
| Berlin | 13. August 2008 | Boeker, Ruth |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 08 15 2815

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-08-2008

| Im Recherchenbericht angeführtes Patentdokument | Datum der Veröffentlichung | Mitglied(er) der Patentfamilie | Datum der Veröffentlichung |
|---|---|---|---|
| WO 2005123101 A | 29-12-2005 | EP 1761271 A1<br>JP 2008502656 T<br>US 2008095719 A1 | 14-03-2007<br>31-01-2008<br>24-04-2008 |
| WO 2007063087 A | 07-06-2007 | KEINE | |
| WO 2006015675 A | 16-02-2006 | DE 102004037753 A1 | 16-03-2006 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0174320 A
- EP 0389700 A
- JP 7196478 A
- US 4251195 A
- US 6214376 B
- WO 03055587 A
- WO 2004050069 A
- WO 02069992 A
- WO 2005123101 A
- US 5043154 A


### In der Beschreibung aufgeführte Nicht-Patentliteratur


- **J. H. Chung et al.** *J. Invest. Dermatol,* 2001, vol. 117, 1218-1224
- **A. Thibodeau.** *Cosmetics & Toiletries,* 2000, vol. 115 (11), 75-82
- **J. Krutmann.** *Hautarzt,* 2003, vol. 54, 809-817
- **T. Kuboto et al.** *Arch. Oral. Biol.,* 1996, vol. 41, 253-262
- **A. L. Ejeil et al.** *J. Periodontol.,* 2003, vol. 74, 188-195
- **Tjäderhane et al.** *J. Dent. Res.,* 1998, vol. 77, 1622-1629
- **Pashley et al.** *J. Dent. Res.,* 2004, vol. 83, 216-221
- **M. E. Ryan et al.** *Curr. Opin. Periodontal.,* 1996, vol. 3, 85-96
- **R. Gendron et al.** *Clin. Diagn. Lab. Immunol.,* 1999, vol. 6, 437-439
- **F.C.Kull et al.** *Applied Microbiology,* 1961, vol. 9, 538-541
- **D.C.Steinberg.** *Cosmetics & Toiletries,* 2000, vol. 115 (11), 59-62
- **K. Bauer ; D. Garbe ; H. Surburg.** Common Fragrance and Flavor Materials. Wiley-VCH, 2001
- **S. Arctander.** Perfume and Flavor Chemicals. Selbstverlag, 1969, vol. I, II
- **B.J.Walsh et al.** *Analytical Biochemistry,* 1992, vol. 203, 187-190
- **Walsh B.J. ; Thornton S.C. ; Penny R. ; Breit S.N.** *Microplate Reader-Based Quantitation of Collagens: Analytical Biochemistry,* 1992, vol. 203, 187-190
- **Nethery A. ; Lyons J.G. ; O'Grady R.L.** *A Spectrometric Collagenase Assay: Analytical Biochemistry,* 1986, vol. 159, 390-395